# EUROPEAN PATENT SPECIFICATION

(11) **EP 2 575 803 B1**
(45) Date of publication and mention of the grant of the patent: **26.07.2017**
(21) Application number: 11790192.6
(22) Date of filing: 20.05.2011
(51) Int. Cl.: A61K 31/337, A61K 47/42, A61K 9/14, A61P 35/04, A61K 38/38, A61K 9/00, A61K 31/7068, A61K 31/555, A61K 31/282

(54) **METHODS OF TREATING BLADDER CANCER**
VERFAHREN ZUR BEHANDLUNG VON BLASENKREBS
PROCÉDÉ DE TRAITEMENT DU CANCER DE LA VESSIE

(30) Priority: 02.06.2010 US 396800 P; 04.03.2011 US 201161449513 P
(43) Date of publication of application: 10.04.2013
(73) Proprietor: Abraxis BioScience, LLC, Los Angeles, CA 90025 (US)
(72) Inventor: DESAI, Neil, P., Los Angeles CA 90025 (US); SOON-SHIONG, Patrick, Los Angeles CA 90049 (US)
(74) Representative: Bezzubova, Olga
(86) International application number: PCT/US2011/037449
(87) International publication number: WO 2011/153009

(56) References cited:
- WO-A1-2006/089290
- WO-A1-2010/068925
- WO-A1-2011/025838
- WO-A2-2008/060651
- US-A1- 2007 116 774
- US-A1- 2008 255 035
- Sridhar, S.S.: "A phase II study of single agent abraxane as second-line therapy in pateients with advanced urothelial carcinoma", ASCO University J Clin Oncol, vol. 27, no. suppl, abstr e16058 21 April 2009 (2009-04-21), pages 1-2, XP002716416, Retrieved from the Internet: URL:http://meetinglibrary.asco.org/content /33661-65 [retrieved on 2013-11-14]
- BARLOW L J ET AL: "Novel intravesical therapies for non-muscle-invasive bladder cancer refractory to BCG", UROLOGIC ONCOLOGY, ELSEVIER, NEW YORK, NY, US, vol. 28, no. 1, 1 January 2010 (2010-01-01), pages 108-111, XP026820997, ISSN: 1078-1439 [retrieved on 2009-12-28]
- CHIEN AMY J ET AL: "A Phase I Study of a 2-Day Lapatinib Chemosensitization Pulse Preceding Nanoparticle Albumin-Bound Paclitaxel for Advanced Solid Malignancies", CLINICAL CANCER RESEARCH, vol. 15, no. 17, September 2009 (2009-09), pages 5569-5575, XP002716417, ISSN: 1078-0432
- SONPAVDE GURU ET AL: "Treatment of metastatic urothelial cancer: opportunities for drug discovery and development", BJU INTERNATIONAL, vol. 102, no. 9, Part B, November 2008 (2008-11), pages 1354-1360, XP002716418, ISSN: 1464-4096
- THOMAS E STINCHCOMBE ET AL: "Phase I and pharmacokinetic trial of carboplatin and albumin-bound paclitaxel, ABI-007 (Abraxane(R)) on three treatment schedules in patients with solid tumors", CANCER CHEMOTHERAPY AND PHARMACOLOGY, SPRINGER, BERLIN, DE, vol. 60, no. 5, 7 February 2007 (2007-02-07), pages 759-766, XP019539690, ISSN: 1432-0843, DOI: 10.1007/S00280-007-0423-X
- KRATZ ET AL: "Albumin as a drug carrier: Design of prodrugs, drug conjugates and nanoparticles", JOURNAL OF CONTROLLED RELEASE, ELSEVIER, AMSTERDAM, NL, vol. 132, no. 3, 18 December 2008 (2008-12-18), pages 171-183, XP025714816, ISSN: 0168-3659, DOI: 10.1016/J.JCONREL.2008.05.010 [retrieved on 2008-11-29]
- WONG J ET AL: "Suspensions for intravenous (IV) injection: A review of development, preclinical and clinical aspects", ADVANCED DRUG DELIVERY REVIEWS, ELSEVIER, vol. 60, no. 8, 22 May 2008 (2008-05-22), pages 939-954, XP022624681, ISSN: 0169-409X, DOI: 10.1016/J.ADDR.2007.11.008 [retrieved on 2008-02-07]

## Description

### CROSS-REFERENCE TO RELATED APPLICATIONS

This application claims the priority benefit of U.S. Provisional Patent Application No. 61/396,800 filed on June 2, 2010 and U.S. Provisional Patent Application No. 61/449,513 filed March 4, 2011.

### TECHNICAL FIELD

The present invention relates to compositions for use in a method of treatment of bladder cancer, wherein compositions comprise nanoparticles that comprise a taxane and an albumin and wherein the method further comprises administering a platinum-based agent and gemcitabine.

### BACKGROUND

Bladder cancer is the fifth most common cancer in North America. Non-muscle-invasive bladder cancer, is difficult to treat and up to 50 percent of patients receiving drugs infused into the bladder (intravesical agents) will experience recurrence of the cancer. Typical second-line treatment for patients with high grade, non-muscle-invasive bladder cancer who have failed standard intravesical therapy is surgical removal of the entire bladder, cystectomy. However many patients are poor surgical candidates or refuse this option. The American Cancer Society estimates that approximately 70,980 people were diagnosed with bladder cancer in the United States in 2009, and that approximately 18,170 died from the disease. The prevalence of bladder cancer in the United States exceeds 500,000 people.

Metastatic bladder cancers are treated with platinum-based agents. Despite initial sensitivity to cisplatin-based chemotherapy regimens, long term control rates of advanced or metastatic disease remain less than 5%. There is currently no standard second-line chemotherapy for metastatic urothelial cancer previously treated with a platinum-based regimen. In this setting, paclitaxel and docetaxel are commonly used despite overall response rates of less than 20%.

Recurrence rate of bladder cancer also presents an additional challenge for bladder cancer treatment. For example, up to 50% of patients treated with BCG (bacillus Calmette-Guerin) for non-muscle-invasive bladder cancer will experience a recurrence within five years. Repeated courses of BCG treatment lead to up to 80% failure rates. Response rates to current second line intravesical therapies average less than 20%.

Taxanes (such as paclitaxel and docetaxel) are a class of diterpenoid drugs that have anti-tumor activity against a wide range of human cancers. Paclitaxel was originally isolated from the bark of the Yew tree, and was known to act by interfering with the normal function of microtubule breakdown. Paclitaxel binds to the β subunit of tubulin, the building blocks of microtubules, causing hyper-stabilization of the microtubule structures. The resulting paclitaxel/microtubule structure is unable to disassemble, thereby arresting mitosis and inhibiting angiogenesis.

Albumin-based nanoparticle compositions have been developed as a drug delivery system for delivering substantially water insoluble drugs such as taxanes. *See,* for example, U.S. Pat. Nos. 5,916,596; 6,506,405; 6,749,868, and 6,537,579 and also in U.S. Pat. Pub. Nos. 2005/0004002 and 2007/0082838. The albumin-based nanoparticle technology utilizes the natural properties of the protein albumin to transport and deliver substantially water insoluble drugs to the site of disease. These nanoparticles are readily incorporated into the body's own transport processes and are able to exploit the tumors' attraction to albumin, enabling the delivery of higher concentrations of the active drug in the nanoparticles to the target site. In addition, the albumin-based nanoparticle technology offers the ability to improve a drug's solubility by avoiding the need for toxic chemicals, such as solvents, in the administration process, thus potentially improving safety through the elimination of solvent-related side effects.

### BRIEF SUMMARY OF THE INVENTION

The description generally relates to a method of treating bladder cancer in an individual in need thereof, comprising administering to the individual an effective amount of a composition comprising nanoparticles comprising a taxane and an albumin (hereinafter also referred to as "the nanoparticle composition" or "taxane nanoparticle composition"). In some examples, the taxane is paclitaxel. In some examples the taxane is docetaxel. In some examples, the albumin is human serum albumin. In some embodiments, the nanoparticles comprise paclitaxel (or docetaxel) coated with albumin. In some examples, the average particle size of the nanoparticles in the nanoparticle composition is no more than about 200 nm (such as less than about 200 nm). In some examples, the composition comprises the albumin stabilized nanoparticle formulation of paclitaxel (*Nab*-paclitaxel (Abraxane®)). In some examples, the composition is *Nab-*paclitaxel (Abraxane®).

Thus, for example, described herein is a method of treating bladder cancer in an individual in need thereof, comprising administering to the individual an effective amount of a composition comprising nanoparticles comprising a taxane and an albumin, wherein the taxane is coated with the albumin. Specifically, described herein is a method of treating bladder cancer in an individual in need thereof, comprising administering to the individual an effective amount of a composition comprising nanoparticles comprising a taxane and an albumin, wherein the average particle size of the nanoparticles in the nanoparticle composition is no greater than about 200 nm (such as less than about 200nm). Specifically, described herein is a method of treating bladder cancer in an individual in need thereof, comprising administering to the individual an effective amount of a composition comprising nanoparticles comprising a taxane and an albumin, wherein the taxane is coated with the albumin, and wherein the average particle size of the nanoparticles in the nanoparticle composition is no greater than about 200 nm (such as less than about 200nm). Specifically described herein is a method of treating bladder cancer in an individual in need thereof, comprising administering to the individual an effective amount of a composition comprising *Nab*-paclitaxel. Specifically, described herein is a method of treating bladder cancer in an individual in need thereof, comprising administering to the individual an effective amount of *Nab*-paclitaxel.

The composition may be administered intravenously or intravesicularly.

The present invention generally relates to combination therapy methods for treating bladder cancer. Thus, generally disclosed is a method of treating bladder cancer in an individual in need thereof, comprising administering to the individual (a) an effective amount of a composition comprising nanoparticles comprising taxane and albumin; and (b) an effective amount of one other agent. The nanoparticle composition and the other agent can be administered simultaneously or sequentially. The nanoparticle composition and the other agent may be administered concurrently. The taxane may be paclitaxel or docetaxel. The albumin may be human serum albumin. The nanoparticles comprise paclitaxel (or docetaxel) may be coated with albumin. The average particle size of the nanoparticles in the nanoparticle composition may be no more than about 200 nm. The composition may comprise the albumin stabilized nanoparticle formulation of paclitaxel (*Nab*-paclitaxel (Abraxane®)). The composition may be *Nab-*paclitaxel (Abraxane®).

Also described herein is a method of treating bladder cancer in an individual in need thereof, comprising administering to the individual (a) an effective amount of a composition comprising nanoparticles comprising taxane and albumin; and (b) an effective amount of a platinum-based agent (such as carboplatin). Alternatively, described herein is a method of treating bladder cancer in an individual in need thereof, comprising administering to the individual (a) an effective amount of a composition comprising nanoparticles comprising taxane and albumin; and (b) an effective amount of an antimetabolite (such as a nucleoside analog, for example gemcitabine). Alternatively, described herein is a method of treating bladder cancer in an individual in need thereof, comprising administering to the individual (a) an effective amount of a composition comprising nanoparticles comprising taxane and albumin; (b) an effective amount of a platinum-based agent (such as carboplatin); and (c) an effective amount of an antimetabolite (such as a nucleoside analog, for example gemcitabine). Alternatively, described herein is a method of treating bladder cancer in an individual in need thereof, comprising administering to the individual (a) an effective amount of a composition comprising nanoparticles comprising paclitaxel coated with albumin (such as Abraxane®); (b) an effective amount of carboplatin; and (c) an effective amount of gemcitabine. In some instances, the method may be carried out in a neoadjuvant setting.

The present invention provides a composition comprising nanoparticles comprising a taxane and albumin for use in a method of treating bladder cancer in an individual, wherein the method further comprises administering a platinum-based agent and gemcitabine.

In some embodiments, the method comprises concurrent administration of the composition, the platinum-based agent and gemcitabine to the individual.

In some embodiments, the bladder cancer is platinum-refractory bladder cancer.

In some embodiments, the bladder cancer is locally advanced bladder cancer.

In some embodiments, the bladder cancer is muscle-invasive bladder cancer.

In some embodiments, the bladder cancer is urothelial carcinoma.

In some embodiments, the bladder cancer is a high grade bladder cancer.

In some embodiments, the bladder cancer is metastatic bladder cancer.

In some embodiments, the platinum-based agent is carboplatin.

In some embodiments, the method comprises administering the carboplatin intravenously at AUC of about 5.

In some embodiments, the method comprises administering gemcitabine intravenously at about 800 mg/m².

In some embodiments, the method comprises administering the nanoparticle composition intravenously.

In some embodiments, the nanoparticle composition is administered at about 260-300 mg/m².

In some embodiments, the taxane is paclitaxel.

In some embodiments, the nanoparticles in the composition have an average diameter of no greater than about 200 nm, preferably less than about 200 nm.

In some embodiments, the taxane in the nanoparticles is coated with albumin.

Bladder cancer that can be treated with methods described herein include, but are not limited to, metastatic bladder cancer, non-muscle-invasive bladder cancer, or bladder cancer that is refractory to a standard therapy (such as BCG) or recurrent after the standard therapy. In some embodiments, the bladder cancer is BCG-refractory non-muscle-invasive bladder cancer. In some embodiments, the bladder cancer is platinum-refractory bladder cancer. In some embodiments, the bladder cancer is platinum-refractory metastatic urothelial carcinoma. In some embodiments, the treatment is first line treatment. In some embodiments, the treatment is second line treatment.

In some embodiments, a method of treating bladder cancer in an individual in need thereof comprises intravesicularly administering to the individual an effective amount of a composition comprising nanoparticles comprising a taxane and an albumin. In some embodiments, a method of treating non-muscle-invasive bladder cancer in an individual in need thereof comprises intravesicularly administering to the individual an effective amount of a composition comprising nanoparticles comprising a taxane and an albumin. In some embodiments, the individual has progressed from an earlier therapy for bladder cancer. In some embodiments, the individual is refractory to an earlier therapy for bladder cancer. In some embodiments, the individual has recurrent bladder cancer. In some embodiments, a method of treating a BCG-refractory non-muscle-invasive bladder cancer in an individual in need thereof comprises intravesicularly administering to the individual an effective amount of a composition comprising nanoparticles comprising a taxane and an albumin. In some embodiments, the amount of the nanoparticle composition is about 100 mg to about 600 mg, including for example about 150 to about 500 mg (such as about 500 mg). In some embodiments, the nanoparticle composition is administered weekly.

In some embodiments, a method of treating bladder cancer in an individual in need thereof comprises intravenously administering to the individual an effective amount of a composition comprising nanoparticles comprising a taxane and an albumin. In some embodiments, a method of treating metastatic bladder cancer (such as metastatic urothelial carcinoma) in an individual in need thereof comprises intravenously administering to the individual an effective amount of a composition comprising nanoparticles comprising a taxane and an albumin. In some embodiments, the bladder cancer is platinum-refractory bladder cancer. In some embodiments, the bladder cancer is platinum-refractory metastatic urothelial carcinoma. In some embodiments, the amount of the nanoparticle composition is about 150 to about 350 mg/m², such as about 260 to about 300 mg/m². In some embodiments, the nanoparticle composition is administered once every three weeks.

The methods described herein can be used for any one or more of the following purposes: alleviating one or more symptoms of bladder cancer, delaying progressing of bladder cancer, shrinking tumor size in bladder cancer patient, inhibiting bladder cancer tumor growth, prolonging overall survival, prolonging disease-free survival, prolonging time to bladder disease progression, preventing or delaying bladder cancer metastasis, reducing (such as eradiating) preexisting bladder cancer metastasis, reducing incidence or burden of preexisting bladder cancer metastasis, preventing recurrence of bladder cancer.

Also described herein are compositions (such as pharmaceutical compositions), medicine, kits, and unit dosages useful for methods described herein.

Described herein is a method of treating bladder cancer in an individual in need thereof, comprising administering to the individual an effective amount of a composition comprising nanoparticles comprising a taxane and an albumin. Specifically, the bladder cancer may be non-muscle-invasive bladder cancer. Specifically, the bladder cancer may be refractory to treatment with BCG, mitomycin C, or interferon. In some instances according to any of the alternatives in the present paragraph, the nanoparticle composition may be administered intravesicularly. In some instances according to any alternatives in the present paragraph, the nanoparticle composition may be administered at the dose of about 150 mg to about 500 mg. In some instances according to any alternatives in the present paragraph, the nanoparticle composition may be administered once weekly.

Described herein is a method of treating platinum-refractory bladder cancer bladder cancer (such as platinum-refractory metastatic bladder cancer) in an individual in need thereof, comprising administering to the individual an effective amount of a composition comprising nanoparticles comprising a taxane and an albumin. Specifically, the nanoparticle composition may be administered intravenously. In some instances according to any of the alternatives in the present paragraph, the nanoparticle composition may be administered at the dose of about 260 mg/m² to about 300 mg/m². In some instances according to any of the alternatives in the present paragraph, the nanoparticle composition may be administered once every three weeks.

According to the invention, the a method of treating bladder cancer in an individual comprises concurrently administering to the individual (a) an effective amount of a composition comprising nanoparticles comprising a taxane and albumin; (b) an effective amount of a platinum-based agent; and (c) an effective amount of an antimetabolite gemcitabine. In some embodiments, the bladder cancer is platinum-refractory bladder cancer. In some embodiments according to any of the embodiments in the present paragraph, the cancer may be locally advanced bladder cancer. In some embodiments according to any of the embodiments in the present paragraph, the cancer is muscle-invasive bladder cancer. In some embodiments according to any of the embodiments in the present paragraph, the nanoparticle composition is administered intravenously. In some embodiments according to any of the embodiments in the present paragraph, the nanoparticle composition is administered at about 260-300 mg/m². In some embodiments according to any of the embodiments in the present paragraph, the platinum-based agent is carboplatin. In some embodiments according to any of the embodiments in the present paragraph, the carboplatin is administered intravenously at AUC of about 5. In some embodiments according to any of the embodiments in the present paragraph, the gemcitabine is administered intravenously at about 800 mg/m².

In some embodiments according to any one of the embodiments in the above three paragraphs, the taxane is paclitaxel.

In some embodiments according to any one of the embodiments in the above two paragraphs, the nanoparticles in the composition have an average diameter of no greater than about 200 nm (such as an average diameter of less than about 200 nm). In some embodiments according to any of the embodiments in the present paragraph, the taxane in the nanoparticles are coated with albumin. In some embodiments according to any of the embodiments in the present paragraph, the bladder cancer is urothelial carcinoma.

In some embodiments according to any one of the embodiments in the above three paragraph, the bladder cancer is a high grade bladder cancer.

These and other aspects and advantages of the present invention will become apparent from the subsequent detailed description and the appended claims. It is to be understood that one, some, or all of the properties of the various embodiments described herein may be combined to form other embodiments of the present invention

### BRIEF DESCRIPTION OF FIGURES

Figure 1 shows the percentage of change in tumor size in 45 platinum-refractory bladder cancer patients treated with Abraxane®.
Figure 2 shows Kaplan Meier progression-free survival graphs for platinum-refractory bladder cancer patients treated with Abraxane®.
Figure 3 shows Kaplan Meier overall survival graphs for platinum-refractory bladder cancer patients treated with Abraxane®.
Figure 4 shows the effect of prognostic factors influencing overall survival for platinum-refractory bladder cancer patients treated with Abraxane®.

### DETAILED DESCRIPTION OF THE INVENTION

The present invention provides compositions comprising nanoparticles comprising a taxane and an albumin, for use in a method of treatingbladder cancer, wherein the method further comprises administering a platinum-based agent and gemcitabine.

We have found that a composition comprising nanoparticles comprising albumin and a taxane, namely, *Nab*-paclitaxel, is highly effective in treating bladder cancer. For example, in a study of 18 patients who previously failed treatment with Bacillus Calmette-Guerin (BCG), 28% were found to demonstrate a complete response after 12 weeks of treatment with *Nab*-paclitaxel by intravesical administration. In a phase II study of *Nab*-paclitaxel in treating platinum-refractory second-line metastatic urothelial carcinoma, *Nab*-paclitaxel was shown to produce a response rate of 33% and a clinical benefit of 58%, respresenting one of the highest reported response rate to date is a second line UC setting. *Nab*-paclitaxel also showed an overall response rate of 44% (13/29) and a disease control rate of 76% (22/29) in a phase II study of intravenously administered *Nab*-paclitaxel as second-line therapy in patients with metastatic urothelial carcinoma. *Nab*-paclitaxel is thus particularly useful for treating bladder cancer, including both non-invasive and mestastatic bladder cancers.

Accordingly, the present application provides a composition comprising nanoparticles comprising a taxane and an albumin for use in a method of treating bladder cancer in an individual in need thereof, wherein the method further comprises administering a platinum-based agent and gemcitabine.

Also contemplated are compositions (such as pharmaceutical compositions), medicine, kits, and unit dosages useful for the methods described herein.

### Definitions

As used herein, "treatment" or "treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread (e.g., metastasis) of the disease, preventing or delaying the recurrence of the disease, reducing recurrence rate of the disease, delay or slowing the progression of the disease, ameliorating the disease state, providing a remission (partial or total) of the disease, decreasing the dose of one or more other medications required to treat the disease, delaying the progression of the disease, increasing the quality of life, and/or prolonging survival. Also encompassed by "treatment" is a reduction of pathological consequence of bladder cancer. The methods of the invention contemplate any one or more of these aspects of treatment.

The term "individual" refers to a mammal and includes, but is not limited to, human, bovine, horse, feline, canine, rodent, or primate.

As used herein, an "at risk" individual is an individual who is at risk of developing bladder cancer. An individual "at risk" may or may not have detectable disease, and may or may not have displayed detectable disease prior to the treatment methods described herein. "At risk" denotes that an individual has one or more so-called risk factors, which are measurable parameters that correlate with development of bladder cancer, which are described herein. An individual having one or more of these risk factors has a higher probability of developing cancer than an individual without these risk factor(s).

"Adjuvant setting" refers to a clinical setting in which an individual has had a history of bladder cancer, and generally (but not necessarily) been responsive to therapy, which includes, but is not limited to, surgery (e.g., surgery resection), radiotherapy, and chemotherapy. However, because of their history of bladder cancer, these individuals are considered at risk of development of the disease. Treatment or administration in the "adjuvant setting" refers to a subsequent mode of treatment. The degree of risk (e.g., when an individual in the adjuvant setting is considered as "high risk" or "low risk") depends upon several factors, most usually the extent of disease when first treated.

"Neoadjuvant setting" refers to a clinical setting in which the method is carried out before the primary/definitive therapy.

As used herein, "delaying" the development of bladder cancer means to defer, hinder, slow, retard, stabilize, and/or postpone development of the disease. This delay can be of varying lengths of time, depending on the history of the disease and/or individual being treated. As is evident to one skilled in the art, a sufficient or significant delay can, in effect, encompass prevention, in that the individual does not develop the disease. A method that "delays" development of bladder cancer is a method that reduces probability of disease development in a given time frame and/or reduces the extent of the disease in a given time frame, when compared to not using the method. Such comparisons are typically based on clinical studies, using a statistically significant number of subjects. Bladder cancer development can be detectable using standard methods, including, but not limited to, computerized axial tomography (CAT scan), Magentic Resonance Imaging (MRI), ultrasound, clotting tests, arteriography, biopsy, urine cytology, and cystoscopy. Development may also refer to bladder cancer progression that may be initially undetectable and includes occurrence, recurrence, and onset.

As used herein, by "combination therapy" is meant that a first agent be administered in conjunction with another agent. "In conjunction with" refers to administration of one treatment modality in addition to another treatment modality, such as administration of a nanoparticle composition described herein in addition to administration of the other agent to the same individual. As such, "in conjunction with" refers to administration of one treatment modality before, during, or after delivery of the other treatment modality to the individual.

The term "effective amount" used herein refers to an amount of a compound or composition sufficient to treat a specified disorder, condition or disease such as ameliorate, palliate, lessen, and/or delay one or more of its symptoms. In reference to bladder cancer, an effective amount comprises an amount sufficient to cause a tumor to shrink and/or to decrease the growth rate of the tumor (such as to suppress tumor growth) or to prevent or delay other unwanted cell proliferation in bladder cancer. In some embodiments, an effective amount is an amount sufficient to delay development of bladder cancer. In some embodiments, an effective amount is an amount sufficient to prevent or delay recurrence. In some embodiments, an effective amount is an amount sufficient to reduce recurrence rate in the individual. An effective amount can be administered in one or more administrations. In the case of bladder cancer, the effective amount of the drug or composition may: (i) reduce the number of bladder cancer cells; (ii) reduce tumor size; (iii) inhibit, retard, slow to some extent and preferably stop bladder cancer cell infiltration into peripheral organs; (iv) inhibit (i.e., slow to some extent and preferably stop) tumor metastasis; (v) inhibit tumor growth; (vi) prevent or delay occurrence and/or recurrence of tumor; (vii) reducing recurrence rate of tumor, and/or (viii) relieve to some extent one or more of the symptoms associated with bladder cancer.

The term "simultaneous administration," as used herein, means that a first therapy and second therapy in a combination therapy are administered with a time separation of no more than about 15 minutes, such as no more than about any of 10, 5, or 1 minutes. When the first and second therapies are administered simultaneously, the first and second therapies may be contained in the same composition (e.g., a composition comprising both a first and second therapy) or in separate compositions (e.g., a first therapy in one composition and a second therapy is contained in another composition).

As used herein, the term "sequential administration" means that the first therapy and second therapy in a combination therapy are administered with a time separation of more than about 15 minutes, such as more than about any of 20, 30, 40, 50, 60, or more minutes. Either the first therapy or the second therapy may be administered first. The first and second therapies are contained in separate compositions, which may be contained in the same or different packages or kits.

As used herein, the term "concurrent administration" means that the administration of the first therapy and that of a second therapy in a combination therapy overlap with each other.

As used herein, by "pharmaceutically acceptable" or "pharmacologically compatible" is meant a material that is not biologically or otherwise undesirable, e.g., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug administration.

It is understood that aspect and embodiments of the invention described herein include "consisting" and/or "consisting essentially of" aspects and embodiments.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise.
, wherein the method further comprises administering a platinum-based agent and gemcitabine.

### Methods of treating bladder cancer

The invention provides a composition comprising nanoparticles comprising a taxane and an albumin for use in methods of treating bladder cancer in an individual (e.g., human) wherein the method further comprises administering a platinum-based agent and gemcitabine.

In some embodiments, the method comprises administering to the individual an effective amount of a composition comprising nanoparticles comprising a taxane and an albumin, wherein the taxane in the nanoparticles is coated with the albumin. In some embodiments, the method comprises administering to the individual an effective amount of a composition comprising nanoparticles comprising paclitaxel and an albumin (such as human serum albumin). In some embodiments, the paclitaxel in the nanoparticles is coated with albumin. In some embodiments, the average particle size of the nanoparticles in the composition is no greater than about 200 nm (such as less than about 200 nm). In some embodiments, the composition comprises *Nab*-paclitaxel. In some embodiments, the composition is *Nab-*paclitaxel.

In some embodiments, the bladder cancer is a low grade bladder cancer. In some embodiments, the bladder cancer is a high grade bladder cancer. In some embodiments, the bladder cancer is invasive. In some embodiments, the bladder cancer is non-invasive. In some embodiments, the bladder cancer is non-muscle invasive.

In some embodiments, the bladder cancer is transitional cell carcinoma or urothelial carcinoma (such as metastatic urothelial carcinoma), including, but not limited to, papillary tumors and flat carcinomas. In some embodiments, the bladder cancer is metastatic urothelial carcinoma. In some embodiments, the bladder cancer is urothelial carcinoma of the bladder. In some embodiments, the bladder cancer is urothelial carcinoma of the ureter. In some embodiments, the bladder cancer is urothelial carcinoma of the urethra. In some embodiments, the bladder cancer is urothelial carcinoma of the renal pelvis.

In some embodiments, the bladder cancer is squamous cell carcinoma. In some embodiments, the bladder cancer is non-squamous cell carcinoma. In some embodiments, the bladder cancer is adenocarcinoma. In some embodiments, the bladder cancer is small cell carcinoma.

In some embodiments, the bladder cancer is early stage bladder cancer, non-metastatic bladder cancer, non-invasive bladder cancer, non-muscle-invasive bladder cancer, primary bladder cancer, advanced bladder cancer, locally advanced bladder cancer (such as unresectable locally advanced bladder cancer), metastatic bladder cancer, bladder cancer in remission, progressive bladder cancer, or recurrent bladder cancer. In some embodiments, the bladder cancer is localized resectable, localized unresectable, or unresectable. In some embodiments, the bladder cancer is a high grade, non-muscle-invasive cancer that has been refractory to standard intra-bladder infusion (intravesical) therapy.

The methods within the context of the invention can be used to treat an individual (e.g., human) who has been diagnosed with or is suspected of having bladder cancer. In some embodiments, the individual is human. In some embodiments, the individual is at least about any of 35, 40, 45, 50, 55, 60, 65, 70, 75, 80, or 85 years old. In some embodiments, the individual is male. In some embodiments, the individual is a female. In some embodiments, the individual has refused surgery. In some embodiments, the individual is medically inoperable. In some embodiments, the individual is at a clinical stage of Ta, Tis, T1, T2, T3a, T3b, or T4.

In some embodiments, the individual has recurrent bladder cancer (such as a bladder cancer at the clinical stage of Ta, Tis, T1, T2, T3a, T3b, or T4) after one or more standard therapy. In some embodiments, the standard therapy is BCG. In some embodiments, the standard therapy is mitomycin C. In some embodiments, the standard therapy is interferon. In some embodiments, the standard therapy was administered intravesicularly. In some embodiments, the individual has recurrent bladder cancer (such as bladder cancer at the clinical stage of Ta, Tis, T1, T2, T3a, T3b, or T4) after treatment with a platinum-based agent (such as carboplatin).

In some embodiments, the individual is refractory to one or more standard therapy. In some embodiments, the standard therapy is BCG. In some embodiments, the standard therapy is mitomycin C. In some embodiments, the standard therapy was administered intravesicularly. In some embodiments, the standard therapy is interferon. In some embodiments, the individual has recurrent bladder cancer (such as bladder cancer at the clinical stage of Ta, Tis, T1, T2, T3a, T3b, or T4) after treatment with a platinum-based agent (such as carboplatin).

In some embodiments, the individual has early stage of bladder cancer, non-metastatic bladder cancer, primary bladder cancer, advanced bladder cancer, locally advanced bladder cancer, for example metastatic bladder cancer, bladder cancer in remission, progressive bladder cancer, or recurrent bladder cancer. In some embodiments, the individual is resistant to treatment of bladder cancer with other agents (such as a non-nanoparticle formulation of taxane, e.g., Taxol® or Taxotere®, platinum-based agents, BCG, mitomycin C, or interferon). In some embodiments, the individual is initially responsive to treatment of bladder cancer with other agents (such as a non-nanoparticle formulation of taxane, e.g., Taxol® or Taxotere®, platinum-based agents, or BCG) but has progressed after treatment.

In some embodiments, the individual is a human who exhibits one or more symptoms associated with bladder cancer. In some embodiments, the individual is at an early stage of bladder cancer. In some embodiments, the individual is at an advanced stage of bladder cancer. In some of embodiments, the individual is genetically or otherwise predisposed (e.g., having a risk factor) to developing bladder cancer. In some embodiments, the individuals at risk for bladder cancer include, e.g., those having relatives who have experienced bladder cancer, and those whose risk is determined by analysis of genetic or biochemical markers. In some embodiments, the individual is positive for SPARC expression (for example based on IHC standard). In some embodiments, the individual is negative for SPARC expression. In some embodiments, the individual has a mutation in FGFR2. In some embodiments, the individual has a mutation in p53. In some embodiments, the individual has a mutation in MIB-1. In some embodiments, the individual has a mutation in FEZ1/LZTS1, PTEN, CDKN2A/MTS1/P6, CDKN2B/INK4B/P15, TSC1, DBCCR1, HRAS1, ERBB2, and NF1.

In some embodiments, the individual has a partial or complete monosomy (such as monosomy 9). In some embodiments, the individual has a deletion in chromosome 11p. In some embodiments, the individual has a deletion in chromosome 13q. In some embodiments, the individual has a deletion in chromosome 17p. In some embodiments, the individual has a deletion in chromosome 1p. In some embodiments, the individual as a chromosome loss of 8p12-22.

In some embodiments, the individual overexpresses p73, c-myc, or cyclin D1.

The methods within the context of the invention may be practiced in an adjuvant setting. In some embodiments, the method is practiced in a neoadjuvant setting, i.e., the method may be carried out before the primary/definitive therapy. In some embodiments, the method is used to treat an individual who has previously been treated. Any of the methods of treatment may be used to treat an individual who has not previously been treated. In some embodiments, the method is used as a first line therapy. In some embodiments, the method is used as a second line therapy.

The methods described herein are useful for various aspects of bladder cancer treatment. In some embodiments, a method of inhibiting bladder cancer cell proliferation (such as bladder cancer tumor growth) in an individual comprises administering to the individual an effective amount of a composition comprising nanoparticles comprising a taxane and an albumin. In some embodiments, at least about 10% (including for example at least about any of 20%, 30%, 40%, 60%, 70%, 80%, 90%, or 100%) cell proliferation is inhibited. In some embodiments, the taxane is paclitaxel. In some embodiments, the taxane in the nanoparticle in the composition is administered by intravenous administration.

In some embodiments, a method of inhibiting bladder cancer tumor metastasis in an individual comprises administering to the individual an effective amount of a composition comprising nanoparticles comprising a taxane and an albumin. In some embodiments, at least about 10% (including for example at least about any of 20%, 30%, 40%, Specifically, methods 60%, 70%, 80%, 90%, or 100%) metastasis is inhibited. Specifically, methods of inhibiting metastasis to lymph node are described. Specifically, methods of inhibiting metastasis to the lung are described. In some embodiments, the taxane is paclitaxel. In some embodiments, the taxane in the nanoparticle in the composition is administered by intravenous administration.

Also described herein is a method of reducing (such as eradiating) preexisting bladder cancer tumor metastasis (such as pulmonary metastasis or metastasis to the lymph node) in an individual, comprising administering to the individual an effective amount of a composition comprising nanoparticles comprising a taxane and an albumin. In some instances, at least about 10% (including for example at least about any of 20%, 30%, 40%, 60%, 70%, 80%, 90%, or 100%) of metastasis may be reduced. Specifically, methods of reducing metastasis to lymph node are described. In some instances, methods of reducing metastasis to the lung are described. In some instances, the taxane may be paclitaxel. In some instances, the taxane in the nanoparticle in the composition may be administered by intravenous administration.

In some embodiments, the invention results in reducing incidence or burden of preexisting bladder cancer tumor metastasis (such as pulmonary metastasis or metastasis to the lymph node) in an individual, comprising administering to the individual an effective amount of a composition comprising nanoparticles comprising a taxane and an albumin. In some embodiments, the taxane is paclitaxel. In some embodiments, the taxane in the nanoparticle in the composition is administered by intravenous administration.

In some embodiments, the invention results in reducing bladder cancer tumor size in an individual, comprising administering to the individual an effective amount of a composition comprising nanoparticles comprising a taxane and an albumin. In some embodiments, the tumor size is reduced at least about 10% (including for example at least about any of 20%, 30%, 40%, 60%, 70%, 80%, 90%, or 100%). In some embodiments, the taxane is paclitaxel. In some embodiments, the taxane in the nanoparticle in the composition is administered by intravenous administration.

In some embodiments, the invention results in prolonging time to disease progression of bladder cancer in an individual, comprising administering to the individual an effective amount of a composition comprising nanoparticles comprising a taxane and an albumin. In some embodiments, the method prolongs the time to disease progression by at least any of 1, 2, 3, 4, 5, 6, 7, 8, 9, 10, 11, or 12 weeks. In some embodiments, the taxane is paclitaxel. In some embodiments, the taxane in the nanoparticle in the composition is administered by intravenous administration.

In some embodiments, the invention results in prolonging survival of an individual having bladder cancer, comprising administering to the individual an effective amount of a composition comprising nanoparticles comprising a taxane and an albumin. In some embodiments, the method prolongs the survival of the individual by at least any of 1, 2, 3, 4, 5, 6, 7, 8, 9, 10, 11, 12, 18, or 24 month. In some embodiments, the taxane is paclitaxel. In some embodiments, the taxane in the nanoparticle in the composition is administered by intravenous administration.

In some embodiments, the invention results in alleviating one or more symptoms in an individual having bladder cancer, comprising administering to the individual an effective amount of a composition comprising nanoparticles comprising a taxane and an albumin. In some embodiments, the taxane in the nanoparticle in the composition is administered by intravenous administration.

The methods of administering the composition comprising nanoparticles comprising a taxane and an albumin in some instances are carried out in conjunction with the administration of one other agent.

In some instances, two or more chemotherapeutic agents are administered in addition to the taxane in the nanoparticle composition. These two or more chemotherapeutic agents may (but not necessarily) belong to different classes of chemotherapeutic agents.

Also disclosed are pharmaceutical compositions comprising nanoparticles comprising a taxane and an albumin (such as human serum albumin) for use in any of the methods of treating bladder cancer described herein.

### Dosing and Method of Administering the Nanoparticle Compositions

The dose of the taxane nanoparticle compositions administered to an individual (such as a human) may vary with the particular composition, the mode of administration, and the type of bladder cancer being treated. In some embodiments, the amount of the composition is effective to result in an objective response (such as a partial response or a complete response). In some embodiments, the amount of the taxane nanoparticle composition is sufficient to result in a complete response in the individual. In some embodiments, the amount of the taxane nanoparticle composition is sufficient to result in a partial response in the individual. In some embodiments, the amount of the taxane nanoparticle composition administered (for example when administered alone) is sufficient to produce an overall response rate of more than about any of 20%, 30%, 40%, 50%, 60%, or 64% among a population of individuals treated with the taxane nanoparticle composition. Responses of an individual to the treatment of the methods described herein can be determined, for example, based on RECIST levels, cystoscopy (with or without biopsy), biopsy, cytology, and CT imaging.

In some embodiments, the amount of the taxane nanoparticle composition is sufficient to produce a negative biopsy in the individual. In some embodiments, the amount of the taxane nanoparticle composition is sufficient to produce a response (partial or complete) based on urine cytology. In some embodiments, the amount of the taxane nanoparticle composition is sufficient to produce both a negative biopsy and a response (partial or complete) based on urine cytology.

In some embodiments, the amount of the taxane nanoparticle composition is not sufficient to cause systemic toxicity, such as grade 2, 3, or 4 systemic toxicity, such as hematuria, dysuria, urinary retension, urinary frequency/urgency, or bladder spasm.

In some embodiments, the amount of the composition is sufficient to prolong progress-free survival of the individual. In some embodiments, the amount of the composition is sufficient to prolong overall survival of the individual. In some embodiments, the amount of the composition (for example when administered alone) is sufficient to produce clinical benefit of more than about any of 50%, 60%, 70%, or 77% among a population of individuals treated with the taxane nanoparticle composition.

In some embodiments, the amount of the composition is an amount sufficient to decrease the size of a tumor, decrease the number of cancer cells, or decrease the growth rate of a tumor by at least about any of 10%, 20%, 30%, 40%, 50%, 60%, 70%, 80%, 90%, 95% or 100% compared to the corresponding tumor size, number of bladder cancer cells, or tumor growth rate in the same subject prior to treatment or compared to the corresponding activity in other subjects not receiving the treatment. Standard methods can be used to measure the magnitude of this effect, such as in vitro assays with purified enzyme, cell-based assays, animal models, or human testing.

In some embodiments, the amount of the taxane (e.g. , paclitaxel) in the composition is below the level that induces a toxicological effect (i.e., an effect above a clinically acceptable level of toxicity) or is at a level where a potential side effect can be controlled or tolerated when the composition is administered to the individual.

In some embodiments, the amount of the composition is close to a maximum tolerated dose (MTD) of the composition following the same dosing regime. In some embodiments, the amount of the composition is more than about any of 80%, 90%, 95%, or 98% of the MTD.

In some embodiments, the amount of a taxane (e.g. , paclitaxel) in the composition is included in any of the following ranges: about 0.1 mg to about 1000 mg, about 0.1 mg to about 2.5 mg, about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg, about 500 mg to about 600 mg, about 600 mg to about 700 mg, about 700 mg to about 800 mg, about 800 mg to about 900 mg, or about 900 mg to about 1000 mg. In some embodiments, the amount of a taxane (e.g., paclitaxel) in the effective amount of the composition (e.g., a unit dosage form) is in the range of about 5 mg to about 500 mg, such as about 30 mg to about 300 mg or about 50 mg to about 200 mg. In some embodiments, the amount of a taxane (e.g., paclitaxel) in the effective amount of the composition (e.g., a unit dosage form) is in the range of about 150 mg to about 500 mg, including for example, about 150 mg, about 225 mg, about 250 mg, about 300 mg, about 325 mg, about 350 mg, about 375 mg, about 400 mg, about 425 mg, about 450 mg, about 475 mg, or about 500 mg. In some embodiments, the concentration of the taxane (e.g., paclitaxel) in the composition is dilute (about 0.1 mg/ml) or concentrated (about 100 mg/ml), including for example any of about 0.1 to about 50 mg/ml, about 0.1 to about 20 mg/ml, about 1 to about 10 mg/ml, about 2 mg/ml to about 8 mg/ml, about 4 to about 6 mg/ml, or about 5 mg/ml. In some embodiments, the concentration of the taxane (e.g., paclitaxel) is at least about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of a taxane (e.g. , paclitaxeL) in the nanoparticle composition include, but are not limited to, at least about any of 25 mg/m², 30 mg/m², 50 mg/m², 60 mg/m², 75 mg/m², 80 mg/m², 90 mg/m², 100 mg/m², 120 mg/m², 125 mg/m², 150 mg/m², 160 mg/m², 175 mg/m², 180 mg/m², 200 mg/m², 210 mg/m², 220 mg/m², 250 mg/m², 260 mg/m², 300 mg/m ² 350 mg/m², 400 mg/m², 500 mg/m², 540 mg/m², 750 mg/m², 1000 mg/m², or 1080 mg/m² of a taxane (e.g., paclitaxel). In various embodiments, the composition includes less than about any of 350 mg/m², 300 mg/m², 250 mg/m², 200 mg/m², 150 mg/m², 120 mg/m², 100 mg/m², 90 mg/m², 50 mg/m², or 30 mg/m² of a taxane (e.g., paclitaxel). In some embodiments, the amount of the taxane (e.g., paclitaxel) per administration is less than about any of 25 mg/m², 22 mg/m², 20 mg/m², 18 mg/m², 15 mg/m², 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², or 1 mg/m². In some embodiments, the effective amount of a taxane (e.g., paclitaxel) in the composition is included in any of the following ranges: about 1 to about 5 mg/m², about 5 to about 10 mg/m², about 10 to about 25 mg/m², about 25 to about 50 mg/m², about 50 to about 75 mg/m², about 75 to about 100 mg/m², about 100 to about 125 mg/m², about 125 to about 150 mg/m², about 150 to about 175 mg/m², about 175 to about 200 mg/m², about 200 to about 225 mg/m², about 225 to about 250 mg/m², about 250 to about 300 mg/m², about 300 to about 350 mg/m², or about 350 to about 400 mg/m². In some embodiments, the effective amount of a taxane (e.g., paclitaxel) in the composition is about 5 to about 300 mg/m², such as about 100 to about 150 mg/m², about 120 mg/m², about 130 mg/m², or about 140 mg/m².

In some embodiments of any of the above aspects, the effective amount of a taxane (e.g., paclitaxel) in the composition includes at least about any of 1 mg/kg, 2.5 mg/kg, 3.5 mg/kg, 5 mg/kg, 6.5 mg/kg, 7.5 mg/kg, 10 mg/kg, 15 mg/kg, 20 mg/kg, 25 mg/kg, 30 mg/kg, 35 mg/kg, 40 mg/kg, 45 mg/kg, 50 mg/kg, 55 mg/kg, or 60 mg/kg. In various embodiments, the effective amount of a taxane (e.g., paclitaxel) in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of a taxane (e.g., paclitaxel).

Exemplary dosing frequencies for the administration of the nanoparticle compositions include, but are not limited to, daily, every two days, every three days, every four days, every five days, every six days, weekly without break, three out of four weeks, once every three weeks, once every two weeks, or two out of three weeks. In some embodiments, the composition is administered about once every 2 weeks, once every 3 weeks, once every 4 weeks, once every 6 weeks, or once every 8 weeks. In some embodiments, the composition is administered at least about any of Ix, 2x, 3x, 4x, 5x, 6x, or 7x (i.e., daily) a week. In some embodiments, the intervals between each administration are less than about any of 6 months, 3 months, 1 month, 20 days, 15, days, 14 days, 13 days, 12 days, 11 days, 10 days, 9 days, 8 days, 7 days, 6 days, 5 days, 4 days, 3 days, 2 days, or 1 day. In some embodiments, the intervals between each administration are more than about any of 1 month, 2 months, 3 months, 4 months, 5 months, 6 months, 8 months, or 12 months. In some embodiments, there is no break in the dosing schedule. In some embodiments, the interval between each administration is no more than about a week.

In some embodiments, the dosing frequency is once every two days for one time, two times, three times, four times, five times, six times, seven times, eight times, nine times, ten times, and eleven times. In some embodiments, the dosing frequency is once every two days for five times. In some embodiments, the taxane (e.g., paclitaxel) is administered over a period of at least ten days, wherein the interval between each administration is no more than about two days, and wherein the dose of the taxane (e.g., paclitaxel) at each administration is about 0.25 mg/m² to about 250 mg/m², about 0.25 mg/m² to about 150 mg/m², about 0.25 mg/m² to about 75 mg/m², such as about 0.25 mg/m² to about 25 mg/m², or about 25 mg/m² to about 50 mg/m².

The administration of the composition can be extended over an extended period of time, such as from about a month up to about seven years. In some embodiments, the composition is administered over a period of at least about any of 2, 3, 4, 5, 6, 7, 8, 9, 10, 11, 12, 18, 24, 30, 36, 48, 60, 72, or 84 months.

In some embodiments, the dosage of a taxane (e.g., paclitaxel) in a nanoparticle composition can be in the range of 5-400 mg/m² when given on a 3 week schedule, or 5-250 mg/m² (such as 80-150 mg/m², for example 100-120 mg/m²) when given on a weekly schedule. For example, the amount of a taxane (e.g., paclitaxel) is about 60 to about 300 mg/m² (e.g., about 260 mg/m²) on a three week schedule.

Other exemplary dosing schedules for the administration of the nanoparticle composition (e.g., paclitaxel/albumin nanoparticle composition) include, but are not limited to, 100 mg/m², weekly, without break; 75 mg/m² weekly, 3 out of four weeks; 100 mg/m²,weekly, 3 out of 4 weeks; 125 mg/m², weekly, 3 out of 4 weeks; 125 mg/m², weekly, 2 out of 3 weeks; 130 mg/m², weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break; 20-150 mg/m² twice a week; and 150-250 mg/m² twice a week. The dosing frequency of the composition may be adjusted over the course of the treatment based on the judgment of the administering physician.

In some embodiments, the individual is treated for at least about any of one, two, three, four, five, six, seven, eight, nine, or ten treatment cycles.

The compositions described herein allow infusion of the composition to an individual over an infusion time that is shorter than about 24 hours. For example, in some embodiments, the composition is administered over an infusion period of less than about any of 24 hours, 12 hours, 8 hours, 5 hours, 3 hours, 2 hours, 1 hour, 30 minutes, 20 minutes, or 10 minutes. In some embodiments, the composition is administered over an infusion period of about 30 minutes.

Other exemplary dose of the taxane (in some embodiments paclitaxel) in the nanoparticle composition include, but is not limited to, about any of 50 mg/m², 60 mg/m², 75 mg/m², 80 mg/m², 90 mg/m², 100 mg/m², 120 mg/m², 160 mg/m², 175 mg/m², 200 mg/m², 210 mg/m², 220 mg/m², 260 mg/m², and 300 mg/m². For example, the dosage of paclitaxel in a nanoparticle composition can be in the range of about 100-400 mg/m² when given on a 3 week schedule, or about 50-250 mg/m² when given on a weekly schedule.

The nanoparticle compositions can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal, and transdermal. In some embodiments, sustained continuous release formulation of the composition may be used. In some embodiments, the composition is administered intravenously. In some embodiments, the composition is administered intravesicularly. In some embodiments, the composition is administered intraarterially. In some embodiments, the composition is administered intraperitoneally.

### Examplary embodiments

In the context of the invention, a method of treating bladder cancer (such as urothelial carcinoma) in an individual comprises administering to the individual an effective amount of a composition comprising nanoparticles comprising a taxane and an albumin, wherein the method further comprises administering a platinum-based agent and gemcitabine. Specifically, a method of treating bladder cancer (such as urothelial carcinoma) in an individual comprises administering to the individual an effective amount of a composition comprising nanoparticles comprising a taxane coated with albumin. Specifically, a method of treating bladder cancer (such as urothelial carcinoma) in an individual comprises administering to the individual an effective amount of a composition comprising nanoparticles comprising a taxane and albumin and having an average diameter of no greater than about 200 nm. Specifically, a method of treating bladder cancer (such as urothelial carcinoma) in an individual comprises administering to the individual an effective amount of a composition comprising nanoparticles comprising a taxane coated with albumin and having an average diameter of no greater than about 200 nm. Specifically, a method of treating bladder cancer (such as urothelial carcinoma) in an individual comprises administering to the individual an effective amount of a composition comprising *Nab*-paclitaxel. Specifically, a method of treating bladder cancer (such as urothelial carcinoma) in an individual comprises administering to the individual an effective amount of *Nab*-paclitaxel.

Furthermore, in the context of the invention, a method of treating bladder cancer (such as urothelial carcinoma) in an individual comprises intravesicularly administering to the individual an effective amount of a composition comprising nanoparticles comprising a taxane and an albumin, wherein the method further comprises administering a platinum-based agent and gemcitabine. Specifically, a method of treating bladder cancer (such as urothelial carcinoma) in an individual comprises intravesicularly administering to the individual an effective amount of a composition comprising nanoparticles comprising a taxane coated with albumin. Specifically, a method of treating bladder cancer (such as urothelial carcinoma) in an individual comprises intravesicularly administering to the individual an effective amount of a composition comprising nanoparticles comprising a taxane and albumin and having an average diameter of no greater than about 200 nm. Specifically, a method of treating bladder cancer (such as urothelial carcinoma) in an individual comprises intravesicularly administering to the individual an effective amount of a composition comprising nanoparticles comprising a taxane coated with albumin and having an average diameter of no greater than about 200 nm, wherein the method further comprises administering a platinum-based agent and gemcitabine. Specifically, a method of treating bladder cancer (such as urothelial carcinoma) in an individual comprises intravesicularly administering to the individual an effective amount of a composition comprising *Nab*-paclitaxel. Specifically, a method of treating bladder cancer (such as urothelial carcinoma) in an individual comprises intravesicularly administering to the individual an effective amount of *Nab-*paclitaxel.

Furthermore, in the context of the invention, a method of treating metastatic bladder cancer (such as metastatic urothelial carcinoma) in an individual comprises intravenously administering to the individual an effective amount of a composition comprising nanoparticles comprising a taxane and an albumin, wherein the method further comprises administering a platinum-based agent and gemcitabine. Specifically, a method of treating metastatic bladder cancer (such as metastatic urothelial carcinoma) in an individual comprises intravenously administering to the individual an effective amount of a composition comprising nanoparticles comprising a taxane coated with albumin. Specifically, a method of treating metastatic bladder cancer (such as metastatic urothelial carcinoma) in an individual comprises intravenously administering to the individual an effective amount of a composition comprising nanoparticles comprising a taxane and albumin and having an average diameter of no greater than about 200 nm. Specifically, a method of treating metastatic bladder cancer (such as metastatic urothelial carcinoma) in an individual comprises intravenously administering to the individual an effective amount of a composition comprising nanoparticles comprising a taxane coated with albumin and having an average diameter of no greater than about 200 nm. Specifically, a method of treating metastatic bladder cancer (such as metastatic urothelial carcinoma) in an individual comprises intravenously administering to the individual an effective amount of a composition comprising *Nab*-paclitaxel. Specifically, a method of treating metastatic bladder cancer (such as metastatic urothelial carcinoma) in an individual, comprises intravenously administering to the individual an effective amount of *Nab*-paclitaxel. In some embodiments, the treatment is second line treatment.

Furthermore, in the context of the invention, a method of treating a platinum-refractory bladder cancer (such as metastatic platinum-refractory bladder cancer, for example metastatic platinum-refractory urothelial carcinoma) in an individual, comprises administering (such as intravenously administering) to the individual an effective amount of a composition comprising nanoparticles comprising a taxane and an albumin, wherein the method further comprises administering a platinum-based agent and gemcitabine. Specifically, a method of treating platinum-refractory bladder cancer (such as metastatic platinum-refractory bladder cancer, for example metastatic platinum-refractory urothelial carcinoma) in an individual comprises administering (such as intravenously administering) to the individual an effective amount of a composition comprising nanoparticles comprising a taxane coated with albumin. Specifically, a method of treating platinum-refractory bladder cancer (such as metastatic platinum-refractory bladder cancer, for example metastatic platinum-refractory urothelial carcinoma) in an individual comprises administering (such as intravenously administering) to the individual an effective amount of a composition comprising nanoparticles comprising a taxane and albumin and having an average diameter of no greater than about 200 nm. Specifically, a method of treating platinum-refractory bladder cancer (such as metastatic platinum-refractory bladder cancer, for example metastatic platinum-refractory urothelial carcinoma) in an individual comprises administering (such as intravenously administering) to the individual an effective amount of a composition comprising nanoparticles comprising a taxane coated with albumin and having an average diameter of no greater than about 200 nm. Specifically, a method of treating platinum-refractory bladder cancer (such as metastatic platinum-refractory bladder cancer, for example metastatic platinum-refractory urothelial carcinoma) in an individual comprises administering (such as intravenously administering) to the individual an effective amount of a composition comprising *Nab*-paclitaxel. Specifically, a method of treating platinum-refractory bladder cancer (such as metastatic platinum-refractory bladder cancer, for example metastatic platinum-refractory urothelial carcinoma) in an individual comprises administering (such as intravenously administering) to the individual an effective amount of *Nab*-paclitaxel.

Furthermore, in the context of the invention, a method of treating platinum-refractory bladder cancer (such as metastatic platinum-refractory bladder cancer, for example metastatic platinum-refractory urothelial carcinoma) in an individual, comprises administering to the individual about 100 to about 300 mg/m² (such as about 260 mg/m²) of a composition comprising nanoparticles comprising a taxane and an albumin, wherein the method further comprises administering a platinum-based agent and gemcitabine. Specifically, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of a composition comprising nanoparticles comprising a taxane coated with albumin. Specifically, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of a composition comprising nanoparticles comprising a taxane and albumin and having an average diameter of no greater than about 200 nm. Specifically, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of a composition comprising nanoparticles comprising a taxane coated with albumin and having an average diameter of no greater than about 200 nm. Specifically, a method of treating platinum-refractory bladder cancer (such as metastatic platinum-refractory bladder cancer, for example metastatic platinum-refractory urothelial carcinoma) in an individual comprises administering to the individual about about 100 to about 300 mg/m² (such as about 260 mg/m²) of a composition comprising *Nab*-pacfitaxel. Specifically, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of *Nab*-paclitaxel. In some embodiments, the composition is administered once every three weeks. In some embodiments, the composition is administered three out of four weeks.

Furthermore, in the context of the invention, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of a composition comprising nanoparticles comprising a taxane and an albumin, wherein the composition is administered intravenously once every three weeks, wherein the method further comprises administering a platinum-based agent and gemcitabine. Specifically, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of a composition comprising nanoparticles comprising a taxane coated with albumin, wherein the composition is administered intravenously once every three weeks. Specifically, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of a composition comprising nanoparticles comprising a taxane and albumin and having an average diameter of no greater than about 200 nm, wherein the composition is administered intravenously once every three weeks. Specifically, a method of treating platinum-refractory bladder cancer (such as metastatic platinum-refractory bladder cancer, for example metastatic platinum-refractory urothelial carcinoma) in an individual, comprises administering to the individual about 100 to about 300 mg/m² (such as about 260 mg/m²) of a composition comprising nanoparticles comprising a taxane coated with albumin and having an average diameter of no greater than about 200 nm, wherein the composition is administered intravenously once every three weeks. Specifically, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of a composition comprising *Nab*-paclitaxel, wherein the composition is administered intravenously once every three weeks. Specifically, a method of treating platinum-refractory bladder cancer (such as metastatic platinum-refractory bladder cancer, for example metastatic platinum-refractory urothelial carcinoma) in an individual comprises administering to the individual about 100 to about 300 mg/m² (such as about 260 mg/m²) of *Nab*-paclitaxel, wherein the composition is intravenously administered once every three weeks.

In some embodiments, the invention results in reducing bladder cancer (such as urothelial carcinoma) tumor size in an individual, comprising administering to the individual an effective amount of a composition comprising nanoparticles comprising a taxane and albumin (e.g., *Nab*-paclitaxel), and further comprising administering a platinum-based agent and gemcitabine. In some embodiments, the composition is administered intravesicularly. In some embodiments, the composition is administered intravenously. In some embodiments, the bladder cancer is metastatic bladder cancer. In some embodiments, the bladder cancer is platinum-refractory bladder cancer. In some embodiments, the bladder cancer is metastatic platinum-refractory bladder cancer. In some embodiments, the nanoparticle composition (e.g. *Nab*-paclitaxel) is administered as second line treatment. In some embodiments, about 100 to about 300 mg/m² (such as about 260 mg/m²) of the nanoparticle composition (e.g. *Nab*-paclitaxel) are intravenously administered to the individual. In some embodiments, the invention results in reducing bladder cancer (such as urothelial carcinoma) tumor size in an individual, wherein the bladder cancer is platinum-refractory bladder cancer, comprising intravenously administering to the individual about 100 to about 300 mg/m² (such as about 260 mg/m²) of a composition comprising nanoparticles comprising a taxane and albumin (e.g., *Nab*-paclitaxel), and further comprising administering a platinum-based agent and gemcitabine. In some embodiments, the invention results in reducing bladder cancer (such as urothelial carcinoma) tumor size in an individual, wherein the bladder cancer is platinum-refractory metastatic bladder cancer, comprising intravenously administering to the individual about 100 to about 300 mg/m² (such as about 260 mg/m²) of a composition comprising nanoparticles comprising a taxane and albumin (e.g., *Nab*-paclitaxel), and further comprising administering a platinum-based agent and gemcitabine.

In some embodiments, the invention results in prolonging time to disease progression of bladder cancer (such as urothelial carcinoma) in an individual, comprising administering to the individual an effective amount of a composition comprising nanoparticles comprising a taxane and albumin (e.g., *Nab*-paclitaxel), and further comprising administering a platinum-based agent and gemcitabine. In some embodiments, the composition is administered intravesicularly. In some embodiments, the composition is administered intravenously. In some embodiments, the bladder cancer is metastatic bladder cancer. In some embodiments, the bladder cancer is platinum-refractory bladder cancer. In some embodiments, the bladder cancer is metastatic platinum-refractory bladder cancer. In some embodiments, the nanoparticle composition (e.g. *Nab*-paclitaxel) is administered as second line treatment. In some embodiments, about 100 to about 300 mg/m² (such as about 260 mg/m²) of the nanoparticle composition (e.g. *Nab*-paclitaxel) are intravenously administered to the individual. In some embodiments, the invention results in prolonging time to disease progression of platinum-refractory bladder cancer (such as platinum-refractory urothelial carcinoma) in an individual, comprising intravenously administering to the individual about 100 to about 300 mg/m² (such as about 260 mg/m²) of a composition comprising nanoparticles comprising a taxane and albumin (e.g., *Nab*-paclitaxel), and further comprising administering a platinum-based agent and gemcitabine. In some embodiments, the invention results in prolonging time to disease progression of platinum-refractory metastatic bladder cancer (such as platinum-refractory metastatic urothelial carcinoma) in an individual, comprising intravenously administering to the individual about 100 to about 300 mg/m² (such as about 260 mg/m²) of a composition comprising nanoparticles comprising a taxane and albumin (e.g., *Nab*-paclitaxel), and further comprising administering a platinum-based agent and gemcitabine.

In some embodiments, the invention results in prolonging survival of an individual having bladder cancer (such as urothelial carcinoma), comprising administering to the individual an effective amount of a composition comprising nanoparticles comprising a taxane and albumin (e.g., *Nab*-paclitaxel), and further comprising administering a platinum-based agent and gemcitabine. In some embodiments, the composition is administered intravesicularly. In some embodiments, the composition is administered intravenously. In some embodiments, the bladder cancer is metastatic bladder cancer. In some embodiments, the bladder cancer is platinum-refractory bladder cancer. In some embodiments, the bladder cancer is metastatic platinum-refractory bladder cancer. In some embodiments, the nanoparticle composition (e.g. *Nab*-paclitaxel) is administered as second line treatment. In some embodiments, about 100 to about 300 mg/m² (such as about 260 mg/m²) of the nanoparticle composition (e.g. *Nab*-paclitaxel) are intravenously administered to the individual. In some embodiments, the invention results in prolonging survival in an individual having platinum-refractory bladder cancer (such as platinum-refractory urothelial carcinoma), comprising intravenously administering to the individual about 100 to about 300 mg/m² (such as about 260 mg/m²) of a composition comprising nanoparticles comprising a taxane and albumin (e.g., *Nab*-paclitaxel), and further comprising administering a platinum-based agent and gemcitabine. In some embodiments, the invention results in prolonging survival in an individual having platinum-refractory metastatic bladder cancer (such as platinum-refractory metastatic urothelial carcinoma), comprising intravenously administering to the individual about 100 to about 300 mg/m² (such as about 260 mg/m²) of a composition comprising nanoparticles comprising a taxane and albumin (e.g., *Nab*-paclitaxel), and further comprising administering a platinum-based agent and gemcitabine.

In some embodiments, the invention results in inhibiting bladder cancer (such as urothelial carcinoma) cell proliferation (such as bladder cancer tumor growth) in an individual, comprising administering to the individual an effective amount of a composition comprising nanoparticles comprising a taxane and albumin (e.g., Nab-paclitaxel), and further comprising administering a platinum-based agent and gemcitabine. In some embodiments, the composition is administered intravesicularly. In some embodiments, the composition is administered intravenously. In some embodiments, the bladder cancer is metastatic bladder cancer. In some embodiments, the bladder cancer is platinum-refractory bladder cancer. In some embodiments, the bladder cancer is metastatic platinum-refractory bladder cancer. In some embodiments, the nanoparticle composition (e.g. Nab-paclitaxel) is administered as second line treatment. In some embodiments, about 100 to about 300 mg/m2 (such as about 260 mg/m2) of the nanoparticle composition (e.g. Nab-paclitaxel) are intravenously administered to the individual. In some embodiments, the invention results in inhibiting platinum-refractory bladder cancer (such as platinum-refractory urothelial carcinoma) cell proliferation (such as bladder cancer tumor growth) in an individual, comprising intravenously administering to the individual about 100 to about 300 mg/m2 (such as about 260 mg/m2) of a composition comprising nanoparticles comprising a taxane and albumin (e.g., Nab-paclitaxel), and further comprising administering a platinum-based agent and gemcitabine. In some embodiments, the invention results in inhibiting platinum-refractory metastatic bladder cancer (such as platinum-refractory metastatic urothelial carcinoma) cell proliferation (such as bladder cancer tumor growth) in an individual, comprising intravenously administering to the individual about 100 to about 300 mg/m2 (such as about 260 mg/m2) a composition comprising nanoparticles comprising a taxane and albumin (e.g., Nab-paclitaxel), and further comprising administering a platinum-based agent and gemcitabine.

In some embodiments, the invention results in inhibiting bladder cancer (such as urothelial carcinoma) tumor metastasis in an individual, comprising administering to the individual an effective amount of a composition comprising nanoparticles comprising a taxane and albumin (e.g., Nab-paclitaxel), and further comprising administering a platinum-based agent and gemcitabine. In some embodiments, the composition is administered intravesicularly. In some embodiments, the composition is administered intravenously. In some embodiments, the bladder cancer is metastatic platinum-refractory bladder cancer. In some embodiments, the nanoparticle composition (e.g. Nab-paclitaxel) is administered as second line treatment. In some embodiments, about 100 to about 300 mg/m2 (such as about 260 mg/m2) of a composition comprising nanoparticles comprising a taxane and albumin (e.g., Nab-paclitaxel) are intravenously administered to the individual. In some embodiments, the invention results in inhibiting bladder cancer (such as urothelial carcinoma) tumor metastasis in an individual, wherein the bladder cancer is metastatic platinum-refractory bladder cancer, comprising intravenously administering to the individual about 100 to about 300 mg/m2 (such as about 260 mg/m2) of a composition comprising nanoparticles comprising a taxane and albumin (e.g., Nab-paclitaxel), and further comprising administering a platinum-based agent and gemcitabine.

In some embodiments, the invention results in reducing (such as eradiating) pre-existing bladder cancer (such as urothelial carcinoma) tumor metastasis (such as pulmonary metastasis or metastasis to the lymph node) in an individual, comprising administering to the individual an effective amount of a composition comprising nanoparticles comprising a taxane and albumin (e.g., Nab-paclitaxel), and further comprising administering a platinum-based agent and gemcitabine. In some embodiments, the composition is administered intravesicularly. In some embodiments, the composition is administered intravenously. In some embodiments, the bladder cancer is metastatic platinum-refractory bladder cancer. In some embodiments, the nanoparticle composition (e.g. Nab-paclitaxel) is administered as second line treatment. In some embodiments, about 100 to about 300 mg/m2 (such as about 260 mg/m2) of the nanoparticle composition (e.g. Nab-paclitaxel) are intravenously administered to the individual. In some embodiments, the invention results in reducing (such as eradiating) pre-existing bladder cancer (such as urothelial carcinoma) tumor metastasis (such as pulmonary metastasis or metastasis to the lymph node) in an individual, wherein the bladder cancer is metastatic platinum-refractory bladder cancer, comprising intravenously administering to the individual about 100 to about 300 mg/m2 (such as about 260 mg/m2) of a composition comprising nanoparticles comprising a taxane and albumin (e.g., Nab-paclitaxel), and further comprising administering a platinum-based agent and gemcitabine.

In some embodiments, the invention results in reducing incidence or burden of preexisting bladder cancer (such as urothelial carcinoma) tumor metastasis (such as pulmonary metastasis or metastasis to the lymph node) in an individual, comprising administering to the individual an effective amount of a composition comprising nanoparticles comprising a taxane and albumin (e.g., Nab-paclitaxel), and further comprising administering a platinum-based agent and gemcitabine. In some embodiments, the composition is administered intravesicularly. In some embodiments, the composition is administered intravenously. In some embodiments, the bladder cancer is metastatic platinum-refractory bladder cancer. In some embodiments, the nanoparticle composition (e.g. Nab-paclitaxel) is administered as second line treatment. In some embodiments, about 100 to about 300 mg/m2 (such as about 260 mg/m2) of the nanoparticle composition (e.g. Nab-paclitaxel) are intravenously administered to the individual. In some embodiments, the invention results in reducing incidence or burden of preexisting bladder cancer (such as urothelial carcinoma) tumor metastasis (such as pulmonary metastasis or metastasis to the lymph node) in an individual, wherein the bladder cancer is metastatic platinum-refractory bladder cancer, comprising intravenously administering to the individual about 100 to about 300 mg/m2 (such as about 260 mg/m2) of a composition comprising nanoparticles comprising a taxane and albumin (e.g., Nab-paclitaxel), and further comprising administering a platinum-based agent and gemcitabine.

### Modes of Administration of Combination Therapies

The dosing regimens described in the section above apply to both monotherapy and combination therapy settings. The modes of administration for combination therapy methods are further described below.

In some embodiments, the nanoparticle composition and the other agent are administered simultaneously. When the drugs are administered simultaneously, the drug in the nanoparticles and the other agent may be contained in the same composition (e.g., a composition comprising both the nanoparticles and the other agent) or in separate compositions (e.g., the nanoparticles are contained in one composition and the other agent is contained in another composition).

In some embodiments, the nanoparticle composition and the other agent are administered sequentially. Either the nanoparticle composition or the other agent may be administered first. The nanoparticle composition and the other agent are contained in separate compositions, which may be contained in the same or different packages.

In some embodiments, the administration of the nanoparticle composition and the other agent are concurrent, i.e., the administration period of the nanoparticle composition and that of the other agent overlap with each other. In some embodiments, the administrations of the nanoparticle composition and the other agent are non-concurrent.

Specifically, described herein is a method of treating bladder cancer in an individual in need thereof, comprises administering to the individual (a) an effective amount of a composition comprising nanoparticles comprising taxane and albumin; and (b) an effective amount of a platinum-based agent (such as carboplatin). Specifically, described herein is a method of treating bladder cancer in an individual in need thereof comprises administering to the individual (a) an effective amount of a composition comprising nanoparticles comprising taxane and albumin: and (b) an effective amount of a platinum-based agent (such as carboplatin). In some instances, the administrations of the nanoparticle composition and the platinum-based agent may be concurrent. In some instances, the method may be being carried out in a neoadjuvant setting.

Specifically, described herein is a method of treating bladder cancer in an individual in need thereof comprises administering to the individual (a) an effective amount of a composition comprising nanoparticles comprising taxane and albumin; and (b) an effective amount of an antimetabolite (such as a nucleoside analog, for example gemcitabine). Specifically, described herein is a method of treating bladder cancer in an individual in need thereof, comprising administering to the individual (a) an effective amount of a composition comprising nanoparticles comprising paclitaxel coated with albumin (such as Abraxane®); and (b) an effective amount of an antimetabolite (such as a nucleoside analog, for example gemcitabine). In some instances, the administrations of the nanoparticle composition and the antimetabolite may be concurrent. In some instances, the method may be being carried out in a neoadjuvant setting.

Specifically, described herein is a method of treating bladder cancer in an individual in need thereof comprises administering to the individual (a) an effective amount of a composition comprising nanoparticles comprising taxane and albumin; (b) an effective amount of a platinum-based agent (such as carboplatin); and (c) an effective amount of an antimetabolite (such as a nucleoside analog, for example gemcitabine). In some instances, the administrations of the nanoparticle composition, the platinum-based agent, and the antimetabolite may be concurrent. In some instances, the method may be carried out in a neoadjuvant setting.

According to the invention, treating bladder cancer in an individual in need thereof comprises administering to the individual (a) an effective amount of a composition comprising nanoparticles comprising paclitaxel coated with albumin (such as Abraxane®); (b) an effective amount of carboplatin; and (c) an effective amount of gemcitabine. In some embodiments, the method is carried out in a neoadjuvant setting. In some embodiments, the administrations of the nanoparticle composition, the carboplatin, and the gemcitabine are concurrent.

In some embodiments, a method of treating bladder cancer (such as urothelial carcinoma) in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine. In some embodiments, a method of treating bladder cancer (such as urothelial carcinoma) in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine wherein the method is carried out in a neoadjuvant setting. In some embodiments, a method of treating bladder cancer (such as urothelial carcinoma) in an individual in need thereof comprises intravenously administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine. In some embodiments, a method of treating bladder cancer (such as urothelial carcinoma) in an individual in need thereof comprises intravenously administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine, wherein the method is carried out in a neoadjuvant setting. In some embodiments, a method of treating bladder cancer (such as urothelial carcinoma) in an individual in need thereof comprises administering (e.g. concurrently administering) to the individual (a) about 260 mg/m2 of a composition comprising nanoparticles comprising paclitaxel coated with albumin (such as Abraxane®); (b) carboplatin at AUC of about 5-6; and (c) about 800-1000 mg/m2 gemcitabine. In some embodiments, a method of treating bladder cancer (such as urothelial carcinoma) in an individual in need thereof comprises administering (e.g. concurrently administering) to the individual (a) about 260 mg/m2 of a composition comprising nanoparticles comprising paclitaxel coated with albumin (such as Abraxane®); (b) carboplatin at AUC of about 5-6; and (c) about 800-1000 mg/m2 gemcitabine, wherein the method is carried out in a neoadjuvant setting.

In some embodiments, a method of treating locally advanced bladder cancer (such as locally advanced urothelial carcinoma) in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine. In some embodiments, a method of treating locally advanced bladder cancer (such as locally advanced urothelial carcinoma) in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine wherein the method is carried out in a neoadjuvant setting. In some embodiments, a method of treating locally advanced bladder cancer (such as locally advanced urothelial carcinoma) in an individual in need thereof comprises intravenously administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine. In some embodiments, a method of treating locally advanced bladder cancer (such as locally advanced urothelial carcinoma) in an individual in need thereof comprises intravenously administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine, wherein the method is carried out in a neoadjuvant setting. In some embodiments, a method of treating locally advanced bladder cancer (such as locally advanced urothelial carcinoma) in an individual in need thereof, comprises administering (e.g. concurrently administering) to the individual (a) about 260 mg/m2 of a composition comprising nanoparticles comprising paclitaxel coated with albumin (such as Abraxane®); (b) carboplatin at AUC of about 5-6; and (c) about 800-1000 mg/m2 gemcitabine. In some embodiments, a method of treating locally advanced bladder cancer (such as locally advanced urothelial carcinoma) in an individual in need thereof comprises administering (e.g. concurrently administering) to the individual (a) about 260 mg/m2 of a composition comprising nanoparticles comprising paclitaxel coated with albumin (such as Abraxane®); (b) carboplatin at AUC of about 5-6; and (c) about 800-1000 mg/m2 gemcitabine, wherein the method is carried out in a neoadjuvant setting.

In some embodiments, a method of treating platinum-refractory bladder cancer (such as platinum-refractory urothelial carcinoma) in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine. In some embodiments, a method of treating platinum-refractory bladder cancer (such as platinum-refractory urothelial carcinoma) in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine wherein the method is carried out in a neoadjuvant setting. In some embodiments, a method of treating platinum-refractory bladder cancer (such as platinum-refractory urothelial (carcinoma) in an individual in need thereof comprises intravenously administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine. In some embodiments, a method of treating platinum-refractory bladder cancer (such as platinum-refractory urothelial carcinoma) in an individual in need thereof comprises intravenously administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine, wherein the method is carried out in a neoadjuvant setting. In some embodiments, a method of treating platinum-refractory bladder cancer (such as platinum-refractory urothelial carcinoma) in an individual in need thereof comprises administering (e.g. concurrently administering) to the individual (a) about 260 mg/m2 of a composition comprising nanoparticles comprising paclitaxel coated with albumin (such as Abraxane®); (b) carboplatin at AUC of about 5-6; and (c) about 800-1000 mg/m2 gemcitabine. In some embodiments, a method of treating platinum-refractory bladder cancer (such as platinum-refractory urothelial carcinoma) in an individual in need thereof comprises administering (e.g. concurrently administering) to the individual (a) about 260 mg/m2 of a composition comprising nanoparticles comprising paclitaxel coated with albumin (such as Abraxane®); (b) carboplatin at AUC of about 5-6; and (c) about 800-1000 mg/m2 gemcitabine, wherein the method is carried out in a neoadjuvant setting.

In some embodiments, a method of treating muscle-invasive bladder cancer (such as muscle-invasive urothelial carcinoma) in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine. In some embodiments, a method of treating muscle-invasive bladder cancer (such as muscle-invasive urothelial carcinoma) in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine, wherein the method is carried out in a neoadjuvant setting. In some embodiments, a method of treating muscle-invasive bladder cancer (such as muscle-invasive urothelial carcinoma) in an individual in need thereof comprises intravenously administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine. In some embodiments, a method of treating muscle-invasive bladder cancer (such as muscle-invasive urothelial carcinoma) in an individual in need thereof comprises intravenously administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine, wherein the method is carried out in a neoadjuvant setting. In some embodiments, a method of treating muscle-invasive bladder cancer (such as muscle-invasive urothelial carcinoma) in an individual in need thereof comprises administering (e.g. concurrently administering) to the individual (a) about 260 mg/m2 of a composition comprising nanoparticles comprising paclitaxel coated with albumin (such as Abraxane®); (b) carboplatin at AUC of about 5-6; and (c) about 800-1000 mg/m2 gemcitabine. In some embodiments, a method of treating muscle-invasive bladder cancer (such as muscle-invasive urothelial carcinoma) in an individual in need thereof comprises administering (e.g. concurrently administering) to the individual (a) about 260 mg/m2 Abraxane®); (b) carboplatin at AUC of about 5-6; and (c) about 800-1000 mg/m2 gemcitabine, wherein the method is carried out in a neoadjuvant setting.

In some embodiments, a method of treating bladder cancer in an individual in need thereof comprises administering (e.g. concurrently administering) to the individual (a) an effective amount of a composition comprising nanoparticles comprising paclitaxel coated with albumin (such as Abraxane®); (b) an effective amount of carboplatin; and (c) an effective amount of gemcitabine, wherein the method is carried out in a neoadjuvant setting. In some embodiments, a method of treating bladder cancer in an individual in need thereof comprises intravenously administering (e.g. concurrently administering) to the individual (a) about 260 mg/m2 Abraxane®; (b) carboplatin at AUC of about 5-6; and (c) about 800-1000 mg/m2 gemcitabine. In some embodiments, a method of treating locally advanced bladder cancer in an individual in need thereof comprises intravenously administering (e.g. concurrently administering) to the individual (a) about 260 mg/m2 Abraxane®; (b) carboplatin at AUC of about 5-6; and (c) about 800-1000 mg/m2 gemcitabine. In some embodiments, a method of treating locally advanced bladder cancer in an individual in need thereof in a neoadjuvant setting comprises intravenously administering (e.g. concurrently administering) to the individual (a) about 260 mg/m2 Abraxane®; (b) carboplatin at AUC of about 5-6; and (c) about 800-1000 mg/m2 gemcitabine. In some embodiments, a method of treating bladder cancer in an individual in need thereof comprises intravenously administering (e.g. concurrently administering) to the individual (a) about 260 mg/m2 Abraxane®; (b) carboplatin at AUC of about 5; and (c) about 800 mg/m2 gemcitabine. In some embodiments, a method of treating locally advanced bladder cancer in an individual in need thereof comprises intravenously administering (e.g. concurrently administering) to the individual (a) about 260 mg/m2 Abraxane®; (b) carboplatin at AUC of about 5; and (c) about 800 mg/m2 gemcitabine. In some embodiments, a method of treating locally advanced bladder cancer in an individual in need thereof in a neoadjuvant setting comprises intravenously administering (e.g. concurrently administering) to the individual (a) about 260 mg/m2 Abraxane®; (b) carboplatin at AUC of about 5; and (c) about 800 mg/m2 gemcitabine. In some embodiments, a method of treating bladder cancer in an individual in need thereof comprises intravenously administering (e.g. concurrently administering) to the individual (a) about 260 mg/m2 Abraxane® on day 1 of each cycle; (b) carboplatin at AUC of about 5-6 on day 1 of each cycle; and (c) about 800-1000 mg/m2 gemcitabine on days 1 and 8 of each cycle. In some embodiments, a method of treating locally advanced bladder cancer in an individual in need thereof comprises intravenously administering (e.g. concurrently administering) to the individual (a) about 260 mg/m2 Abraxane® on day 1 of each cycle; (b) carboplatin at AUC of about 5-6 on day 1 of each cycle; and (c) about 800-1000 mg/m2 gemcitabine on days 1 and 8 of each cycle. In some embodiments, a method of treating locally advanced bladder cancer in an individual in need thereof in a neoadjuvant setting comprises intravenously administering (e.g. concurrently administering) to the individual (a) about 260 mg/m2 Abraxane® on day 1 of each cycle; (b) carboplatin at AUC of about 5-6 on day 1 of each cycle; and (c) about 800-1000 mg/m2 gemcitabine on days 1 and 8 of each cycle.

In some embodiments, a method of treating platinum-refractory bladder cancer in an individual in need thereof comprises intravenously administering (e.g. concurrently administering) to the individual (a) about 260 mg/m2 Abraxane®; (b) carboplatin at AUC of about 5-6; and (c) about 800-1000 mg/m2 gemcitabine. In some embodiments, a method of treating platinum-refractory bladder cancer in an individual in need thereof in a neoadjuvant setting comprises intravenously administering (e.g. concurrently administering) to the individual (a) about 260 mg/m2 Abraxane®; (b) carboplatin at AUC of about 5-6; and (c) about 800-1000 mg/m2 gemcitabine. In some embodiments, a method of treating platinum-refractory bladder cancer in an individual in need thereof comprises intravenously administering (e.g. concurrently administering) to the individual (a) about 260 mg/m2 Abraxane®; (b) carboplatin at AUC of about 5; and (c) about 800 mg/m2 gemcitabine. In some embodiments, a method of treating platinum-refractory bladder cancer in an individual in need thereof in a neoadjuvant settings comprises intravenously administering (e.g., concurrently administering) to the individual (a) about 260 mg/m2 Abraxane®; (b) carboplatin at AUC of about 5; and (c) about 800 mg/m2 gemcitabine. In some embodiments, a method of treating platinum-refractory bladder cancer in an individual in need thereof comprises intravenously administering (e.g. concurrently administering) to the individual (a) about 260 mg/m2 Abraxane® on day 1 of each cycle; (b) carboplatin at AUC of about 5-6 on day 1 of each cycle; and (c) about 800-1000 mg/m2 gemcitabine on days 1 and 8 of each cycle. In some embodiments, a method of treating platinum-refractory bladder cancer in an individual in need thereof in a neoadjuvant setting, comprises intravenously administering (e.g. concurrently administering) to the individual (a) about 260 mg/m2 Abraxane® on day 1 of each cycle; (b) carboplatin at AUC of about 5-6 on day 1 of each cycle; and (c) about 800-1000 mg/m2 gemcitabine on days 1 and 8 of each cycle.

In some embodiments, a method of treating muscle-invasive bladder cancer in an individual in need thereof comprises intravenously administering (e.g. concurrently administering) to the individual (a) about 260 mg/m2 Abraxane®; (b) carboplatin at AUC of about 5-6; and (c) about 800-1000 mg/m2 gemcitabine. In some embodiments, a method of treating muscle-invasive bladder cancer in an individual in need thereof in a neoadjuvant setting comprises intravenously administering (e.g. concurrently administering) to the individual (a) about 260 mg/m2 Abraxane®; (b) carboplatin at AUC of about 5-6; and (c) about 800-1000 mg/m2 gemcitabine. In some embodiments, a method of treating bladder cancer in an individual in need thereof comprises intravenously administering (e.g. concurrently administering) to the individual (a) about 260 mg/m2 Abraxane®; (b) carboplatin at AUC of about 5; and (c) about 800 mg/m2 gemcitabine. In some embodiments, a method of treating muscle-invasive bladder cancer in an individual in need thereof comprises intravenously administering (e.g. concurrently administering) to the individual (a) about 260 mg/m2 Abraxane®; (b) carboplatin at AUC of about 5; and (c) about 800 mg/m2 gemcitabine. In some embodiments, a method of treating muscle-invasive bladder cancer in an individual in need thereof in a neoadjuvant setting comprises intravenously administering (e.g., concurrently administering) to the individual (a) about 260 mg/m2 Abraxane®); (b) carboplatin at AUC of about 5; and (c) about 800 mg/m2 gemcitabine.In some embodiments, a method of treating muscle-invasive bladder cancer in an individual in need thereof comprises intravenously administering (e.g. concurrently administering) to the individual (a) about 260 mg/m2 Abraxane® on day 1 of each cycle; (b) carboplatin at AUC of about 5-6 on day 1 of each cycle; and (c) about 800-1000 mg/m2 gemcitabine on days 1 and 8 of each cycle. In some embodiments, a method of treating muscle invasive bladder cancer in an individual in need thereof in a neoadjuvant setting comprises intravenously administering (e.g. concurrently administering) to the individual (a) about 260 mg/m2 Abraxane® on day 1 of each cycle; (b) carboplatin at AUC of about 5-6 on day 1 of each cycle; and (c) about 800-1000 mg/m2 gemcitabine on days 1 and 8 of each cycle.

In some embodiments, a method of reducing tumor size in an individual with bladder cancer comprises administering (e.g., concurrently administering) to the individual (a) an effective amount of a composition comprising nanoparticles comprising paclitaxel coated with albumin (such as Abraxane®); (b) an effective amount of carboplatin; and (c) an effective amount of gemcitabine, wherein the method is carried out in a neoadjuvant setting. In some embodiments, a method of reducing tumor size in an individual with bladder cancer comprises intravenously administering (e.g., concurrently administering)to the individual (a) about 260 mg/m2 Abraxane®; (b) carboplatin at AUC of about 5-6; and (c) about 800-1000 mg/m2 gemcitabine. In some embodiments, a method of reducing tumor size in an individual with locally advanced bladder cancer comprises intravenously administering (e.g., concurrently administering) to the individual (a) about 260 mg/m2 Abraxane®); (b) carboplatin at AUC of about 5-6; and (c) about 800-1000 mg/m2 gemcitabine. In some embodiments, a method of reducing tumor size in an individual with locally advanced bladder cancer in a neoadjuvant setting comprises intravenously administering (e.g., concurrently administering) to the individual (a) about 260 mg/m2 Abraxane®); (b) carboplatin at AUC of about 5-6; and (c) about 800-1000 mg/m2 gemcitabine. In some embodiments, a method of reducing tumor size in an individual with bladder cancer comprises intravenously administering (e.g., concurrently administering) to the individual (a) about 260 mg/m2 Abraxane®; (b) carboplatin at AUC of about 5; and (c) about 800 mg/m2 gemcitabine. In some embodiments, a method of reducing tumor size in an individual with locally advanced bladder cancer comprises intravenously administering (e.g., concurrently administering) to the individual (a) about 260 mg/m2 Abraxane®; (b) carboplatin at AUC of about 5; and (c) about 800 mg/m2 gemcitabine. In some embodiments, a method of reducing tumor size in an individual with locally advanced bladder cancer in a neoadjuvant setting comprises intravenously administering (e.g., concurrently administering) to the individual (a) about 260 mg/m2 Abraxane®; (b) carboplatin at AUC of about 5; and (c) about 800 mg/m2 gemcitabine.

Also contemplated are pharmaceutical compositions, kits, and medicines comprising the nanoparticle composition, the platinum-based agents, and the antimetabolite. For example, a pharmaceutical composition (or a medicine) for treating bladder cancer may comprise (a) an effective amount of a composition comprising nanoparticles comprising paclitaxel coated with albumin (such as Abraxane®); (b) an effective amount of carboplatin; and (c) an effective amount of gemcitabine. A kit may comprise (a) an effective amount of a composition comprising nanoparticles comprising paclitaxel coated with albumin (such as Abraxane®); (b) an effective amount of carboplatin; and (c) an effective amount of gemcitabine. The kit may further comprise an instruction for treating bladder cancer.

In some embodiments, a pharmaceutical composition (or a medicine) for treating bladder cancer comprises (a) about 260 mg/m2 Abraxane®); (b) carboplatin at AUC of about 5-6; and (c) about 800-1000 mg/m2 gemcitabine. Described herein is also a kit comprising (a) about 260 mg/m2 Abraxane®); (b) carboplatin at AUC of about 5-6; and (c) about 800-1000 mg/m2 gemcitabine. The kit may further comprise an instruction for treating bladder cancer (e.g., locally advanced bladder cancer; locally advanced bladder cancer in a neoadjuvant setting)

### Nanoparticle Compositions

The nanoparticle compositions described herein comprise nanoparticles comprising (in various embodiments consisting essentially of) a taxane (such as paclitaxel) and an albumin ( such as human serum albumin). Nanoparticles of poorly water soluble drugs (such as taxane) have been disclosed in, for example, U.S. Pat. Nos. 5,916,596; 6,506,405; 6,749,868, and 6,537,579 and also in U.S. Pat. Pub. Nos. 2005/0004002, 2006/0263434, and 2007/0082838; PCT Patent Application WO08/137148.

In some embodiments, the composition comprises nanoparticles with an average or mean diameter of no greater than about 1000 nanometers (nm), such as no greater than about any of 900, 800, 700, 600, 500, 400, 300, 200, and 100 nm. In some embodiments, the average or mean diameters of the nanoparticles is no greater than about 200 nm. In some embodiments, the average or mean diameters of the nanoparticles is no greater than about 150 nm. In some embodiments, the average or mean diameters of the nanoparticles is no greater than about 100 nm. In some embodiments, the average or mean diameter of the nanoparticles is about 20 to about 400 nm. In some embodiments, the average or mean diameter of the nanoparticles is about 40 to about 200 nm. In some embodiments, the nanoparticles are sterile-filterable.

In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of the nanoparticles in the composition fall within the range of about 20 to about 400 nm, including for example about 20 to about 200 nm, about 40 to about 200 nm, about 30 to about 180 nm, and any one of about 40 to about 150, about 50 to about 120, and about 60 to about 100 nm.

In some embodiments, the albumin has sulfhydral groups that can form disulfide bonds. In some embodiments, at least about 5% (including for example at least about any one of 10%, 15%, 20%, 25%, 30%, 40%, 50%, 60%, 70%, 80%, or 90%) of the albumin in the nanoparticle portion of the composition are crosslinked (for example crosslinked through one or more disulfide bonds).

In some embodiments, the nanoparticles comprise the taxane (such as paclitaxel) coated with an albumin (e.g., human serum albumin). In some embodiments, the composition comprises taxane in both nanoparticle and non-nanoparticle forms, wherein at least about any one of 50%, 60%, 70%, 80%, 90%, 95%, or 99% of the taxane in the composition are in nanoparticle form. In some embodiments, the taxane in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of taxane that is substantially free of polymeric materials (such as polymeric matrix).

In some embodiments, the composition comprises albumin in both nanoparticle and non-nanoparticle portions of the composition, wherein at least about any one of 50%, 60%, 70%, 80%, 90%, 95%, or 99% of the albumin in the composition are in non-nanoparticle portion of the composition.

In some embodiments, the weight ratio of albumin (such as human serum albumin) and taxane in the nanoparticle composition is about 18:1 or less, such as about 15:1 or less, for example about 10:1 or less. In some embodiments, the weight ratio of albumin (such as human serum albumin) and taxane in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of albumin and taxane in the nanoparticle portion of the composition is about any one of 1:2, 1:3, 1:4, 1:5, 1:10, 1:15, or less. In some embodiments, the weight ratio of the albumin (such as human serum albumin) and the taxane in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 1:1 to about 1:1.

In some embodiments, the nanoparticle composition comprises one or more of the above characteristics.

The nanoparticles described herein may be present in a dry formulation (such as lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

In some embodiments, the pharmaceutically acceptable carrier comprises human serum albumin. Human serum albumin (HSA) is a highly soluble globular protein of Mᵣ 65K and consists of 585 amino acids. HSA is the most abundant protein in the plasma and accounts for 70-80 % of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (see, e.g., Tullis, JAMA, 237, 355-360, 460-463, (1977)) and Houser et al., Surgery, Gynecology and Obstetrics, 150, 811-816 (1980)) and in conjunction with exchange transfusion in the treatment of neonatal hyperbilirubinemia (see, e.g., Finlayson, Seminars in Thrombosis and Hemostasis, 6, 85-120, (1980)). Other albumins are contemplated, such as bovine serum albumin. Use of such non-human albumins could be appropriate, for example, in the context of use of these compositions in non-human mammals, such as the veterinary (including domestic pets and agricultural context).

Human serum albumin (HSA) has multiple hydrophobic binding sites (a total of eight for fatty acids, an endogenous ligand of HSA) and binds a diverse set of taxanes, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, e.g., Fehske et al., Biochem. Pharmcol., 30, 687-92 (198a), Vorum, Dan. Med. Bull., 46, 379-99 (1999), Kragh-Hansen, Dan. Med. Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (199b), and Carter et al., Λdv. Protein. Chem., 45, 153-203 (1994)). Paclitaxel and propofol have been shown to bind HSA (see, e.g., Paal et al., Eur. J. Biochem., 268(7), 2187-91 (200a), Purcell et al., Biochim. Biophys. Acta, 1478(a), 61-8 (2000), Altmayer et al., Arzneimittelforschung, 45, 1053-6 (1995), and Garrido et al., Rev. Esp. Anestestiol. Reanim., 41, 308-12 (1994)). In addition, docetaxel has been shown to bind to human plasma proteins (see, e.g., Urien et al., Invest. New Drugs, 14(b), 147-51 (1996)).

The albumin (such as human serum albumin) in the composition generally serves as a carrier for the taxane, i.e., the albumin in the composition makes the taxane more readily suspendable in an aqueous medium or helps maintain the suspension as compared to compositions not comprising an albumin. This can avoid the use of toxic solvents (or surfactants) for solubilizing the taxane, and thereby can reduce one or more side effects of administration of the taxane into an individual (such as a human). Thus, in some embodiments, the composition described herein is substantially free (such as free) of surfactants, such as Cremophor (including Cremophor EL^{®} (BASF)). In some embodiments, the nanoparticle composition is substantially free (such as free) of surfactants. A composition is "substantially free of Cremophor" or "substantially free of surfactant" if the amount of Cremophor or surfactant in the composition is not sufficient to cause one or more side effect(s) in an individual when the nanoparticle composition is administered to the individual. In some embodiments, the nanoparticle composition contains less than about any one of 20%, 15%, 10%, 7.5%, 5%, 2.5%, or 1% organic solvent or surfactant.

The amount of albumin in the composition described herein will vary depending on other components in the composition. In some embodiments, the composition comprises an albumin in an amount that is sufficient to stabilize the taxane in an aqueous suspension, for example, in the form of a stable colloidal suspension (such as a stable suspension of nanoparticles). In some embodiments, the albumin is in an amount that reduces the sedimentation rate of the taxane in an aqueous medium. For particle-containing compositions, the amount of the albumin also depends on the size and density of nanoparticles of the taxane.

A taxane is "stabilized" in an aqueous suspension if it remains suspended in an aqueous medium (such as without visible precipitation or sedimentation) for an extended period of time, such as for at least about any of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (such as human). Stability of the suspension is generally (but not necessarily) evaluated at a storage temperature (such as room temperature (such as 20-25 °C) or refrigerated conditions (such as 4 °C)). For example, a suspension is stable at a storage temperature if it exhibits no flocculation or particle agglomeration visible to the naked eye or when viewed under the optical microscope at 1000 times, at about fifteen minutes after preparation of the suspension. Stability can also be evaluated under accelerated testing conditions, such as at a temperature that is higher than about 40 °C.

In some embodiments, the albumin is present in an amount that is sufficient to stabilize the taxane in an aqueous suspension at a certain concentration. For example, the concentration of the taxane in the composition is about 0.1 to about 100 mg/ml, including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg /ml. In some embodiments, the concentration of the taxane is at least about any of 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, and 50 mg/ml. In some embodiments, the albumin is present in an amount that avoids use of surfactants (such as Cremophor), so that the composition is free or substantially free of surfactant (such as Cremophor).

In some embodiments, the composition, in liquid form, comprises from about 0.1% to about 50% (w/v) (e.g. about 0.5% (w/v), about 5% (w/v), about 10% (w/v), about 15% (w/v), about 20% (w/v), about 30% (w/v), about 40% (w/v), or about 50% (w/v)) of albumin. In some embodiments, the composition, in liquid form, comprises about 0.5% to about 5% (w/v) of albumin.

In some embodiments, the weight ratio of albumin, e.g., albumin, to the taxane in the nanoparticle composition is such that a sufficient amount of taxane binds to, or is transported by, the cell. While the weight ratio of albumin to taxane will have to be optimized for different albumin and taxane combinations, generally the weight ratio of albumin, e.g., albumin, to taxane (w/w) is about 0.01:1 to about 100:1, about 0.02:1 to about 50:1, about 0.05:1 to about 20:1, about 0.1:1 to about 20:1, about 1:1 to about 18:1, about 2:1 to about 15:1, about 3:1 to about 12:1, about 4:1 to about 10:1, about 5:1 to about 9:1, or about 9:1. In some embodiments, the albumin to taxane weight ratio is about any of 18:1 or less, 15:1 or less, 14:1 or less, 13:1 or less, 12:1 or less, 11:1 or less, 10:1 or less, 9:1 or less, 8:1 or less, 7:1 or less, 6:1 or less, 5:1 or less, 4:1 or less, and 3:1 or less. In some embodiments, the weight ratio of the albumin (such as human serum albumin) and the taxane in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 1:1 to about 1:1.

In some embodiments, the albumin allows the composition to be administered to an individual (such as human) without significant side effects. In some embodiments, the albumin ( such as human serum albumin) is in an amount that is effective to reduce one or more side effects of administration of the taxane to a human. The term "reducing one or more side effects of administration of the taxane" refers to reduction, alleviation, elimination, or avoidance of one or more undesirable effects caused by the taxane, as well as side effects caused by delivery vehicles (such as solvents that render the taxanes suitable for injection) used to deliver the taxane. Such side effects include, for example, myelosuppression, neurotoxicity, hypersensitivity, inflammation, venous irritation, phlebitis, pain, skin irritation, peripheral neuropathy, neutropenic fever, anaphylactic reaction, venous thrombosis, extravasation, and combinations thereof. These side effects, however, are merely exemplary and other side effects, or combination of side effects, associated with taxanes can be reduced.

In some embodiments, the nanoparticle composition comprises Abraxane^{®} (*Nab-*paclitaxel). In some embodiments, the nanoparticle composition is Abraxane^{®} (*Nab*-paclitaxel). Abraxane^{®} is a formulation of paclitaxel stabilized by human albumin USP, which can be dispersed in directly injectable physiological solution. When dispersed in a suitable aqueous medium such as 0.9% sodium chloride injection or 5% dextrose injection, Abraxane^{®} forms a stable colloidal suspension of paclitaxel. The mean particle size of the nanoparticles in the colloidal suspension is about 130 nanometers. Since HSA is freely soluble in water, Abraxane^{®} can be reconstituted in a wide range of concentrations ranging from dilute (0.1 mg/ml paclitaxel) to concentrated (20 mg/ml paclitaxel), including for example about 2 mg/ml to about 8 mg/ml, about 5 mg/ml.

Methods of making nanoparticle compositions are known in the art. For example, nanoparticles containing taxanes (such as paclitaxel) and albumin (such as human serum albumin) can be prepared under conditions of high shear forces (e.g., sonication, high pressure homogenization, or the like). These methods are disclosed in, for example, U.S. Pat. Nos. 5,916,596; 6,506,405; 6,749,868, and 6,537,579 and also in U.S. Pat. Pub. No. 2005/0004002, 2007/0082838, 2006/0263434and PCT Application WO08/137148.

Briefly, the taxane (such as paclitaxel) is dissolved in an organic solvent, and the solution can be added to an albumin solution. The mixture is subjected to high pressure homogenization. The organic solvent can then be removed by evaporation. The dispersion obtained can be further lyophilized. Suitable organic solvent include, for example, ketones, esters, ethers, chlorinated solvents, and other solvents known in the art. For example, the organic solvent can be methylene chloride or chloroform/ethanol (for example with a ratio of 1:9, 1:8, 1:7, 1:6, 1:5, 1:4, 1:3, 1:2, 1:1, 2:1, 3:1, 4:1, 5:1, 6:1, 7:1, 8:1, or 9:1.

### Other Components in the Nanoparticle Compositions

The nanoparticles described herein can be present in a composition that include other agents, excipients, or stabilizers. For example, to increase stability by increasing the negative zeta potential of nanoparticles, certain negatively charged components may be added. Such negatively charged components include, but are not limited to bile salts of bile acids consisting of glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid and others; phospholipids including lecithin (egg yolk) based phospholipids which include the following phosphatidylcholines: palmitoyloleoylphosphatidylcholine, palmitoyllinoleoylphosphatidylcholine, stearoyllinoleoylphosphatidylchofine stearoyloleoylphosphatidylcholine, stearoylarachidoylphosphatidylcholine, and dipalmitoylphosphatidylcholine. Other phospholipids including L-α-dimyristoylphosphatidylcholine (DMPC), dioleoylphosphatidylcholine (DOPC), distearyolphosphatidylcholine (DSPC), hydrogenated soy phosphatidylcholine (HSPC), and other related compounds. Negatively charged surfactants or emulsifiers are also suitable as additives, e.g., sodium cholesteryl sulfate and the like.

In some embodiments, the composition is suitable for administration to a human. In some embodiments, the composition is suitable for administration to a mammal such as, in the veterinary context, domestic pets and agricultural animals. There are a wide variety of suitable formulations of the nanoparticle composition (see, e.g., U.S. Pat. Nos. 5,916,596 and 6,096,331). The following formulations and methods are merely exemplary and are in no way limiting. Formulations suitable for oral administration can consist of (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Examples of suitable carriers, excipients, and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate, and mineral oil. The formulations can additionally include lubricating agents, wetting agents, emulsifying and suspending agents, preserving agents, sweetening agents or flavoring agents.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described. Injectable formulations are preferred.

In some embodiments, the composition is formulated to have a pH range of about 4.5 to about 9.0, including for example pH ranges of any of about 5.0 to about 8.0, about 6.5 to about 7.5, and about 6.5 to about 7.0. In some embodiments, the pH of the composition is formulated to no less than about 6, including for example no less than about any of 6.5, 7, or 8 (such as about 8). The composition can also be made to be isotonic with blood by the addition of a suitable tonicity modifier, such as glycerol.

### Kits, Medicines, and Compositions

Disclosed herein are kits, medicines, compositions, and unit dosage forms for use in any of the methods described herein.

Kits may include one or more containers comprising taxane-containing nanoparticle compositions (or unit dosage forms and/or articles of manufacture) and/or another agent (such as the agents described herein), and, further comprise instructions for use in accordance with any of the methods described herein. The kit may further comprise a description of selection an individual suitable or treatment. Instructions supplied in the kits are typically written instructions on a label or package insert (e.g., a paper sheet included in the kit), but machine-readable instructions (e.g., instructions carried on a magnetic or optical storage disk) are also acceptable.

For example, the kit may comprise a) composition comprising nanoparticles comprising a taxane and an albumin (such as human serum albumin), and b) instructions for administering the nanoparticle composition for treatment of bladder cancer. In some instances, the kit may comprise a) a composition comprising nanoparticles comprising a taxane and an albumin (such as human serum albumin), b) an effective amount of one other agent, wherein the other agent inhibits microtubule disassembly, and c) instructions for administering (such as administering intravesicularly or intravenously) the nanoparticle composition and the other agents for treatment of bladder cancer. The nanoparticles and the other agents can be present in separate containers or in a single container. For example, the kit may comprise one distinct composition or two or more compositions wherein one composition comprises nanoparticles and one composition comprises another agent.

The kits diclosed herein may be in suitable packaging. Suitable packaging include, but is not limited to, vials, bottles, jars, flexible packaging (e.g., seled Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the nanoparticle compositions generally include information as to dosage, dosing schedule, and route of administration for the intended treatment. The containers may be unit doses, bulk packages (e.g., multi-dose packages) or subunit doses. For example, kits may be provided that contain sufficient dosages of the taxane (such as taxane) as disclosed herein to provide effective treatment of an individual for an extended period, such as any of a week, 8 days, 9 days, 10 days, 11 days, 12 days, 13 days, 2 weeks, 3 weeks, 4 weeks, 6 weeks, 8 weeks, 3 months, 4 months, 5 months, 7 months, 8 months, 9 months, or more. Kits may also include multiple unit doses of the taxane and pharmaceutical compositions and instructions for use and packaged in quantities sufficient for storage and use in pharmacies, for example, hospital pharmacies and compounding pharmacies.

Also disclosed herein are medicines, compositions, and unit dosage forms useful for the methods described herein. Specifically, disclosed herein is a medicine (or composition) for use in treating bladder cancer, comprising nanoparticles comprising a taxane and an albumin (such as human serum albumin). Specifically, disclosed herein is a medicine (or composition or a unit dosage form) for use in treating bladder cancer in conjunction with another agent, comprising nanoparticles comprising a taxane and an albumin (such as human serum albumin), wherein the other agent inhibits microtubule disassembly. Specifically, disclosed herein is a medicine (or composition or a unit dosage form) for use in treating bladder cancer, comprising nanoparticles comprising a taxane and an albumin (such as human serum albumin) and one other agent, wherein the other agent inhibits microtubule disassembly.

The invention will now be described in greater detail by reference to the following non-limiting examples. The following examples, as far as directed to the subject-matter defined in the claims, further illustrate the invention.

### EXAMPLES

### Example 1. Phase I trial of intravesicular administration of nanoparticle albumin-bound paclitaxel in the treatment of BCG-refractory non-muscle-invasive bladder cancer

This example demonstrates the activity of *Nab*-paclitaxel in the treatment of non-muscle invasive bladder cancer when administered intravesicularly.

### Eligibility criteria

Inclusion criteria included recurrent high grade (HG) Ta, T1, and Tis transitional cell carcinomas failing at least one prior regimen of standard intravesical therapy (including BCG, mitomycin C, or interferon). The patients are either inoperable due to medical comorbidities or refusal of cystectomy.

### Drug administration

*Nab*-paclitaxel reconstituted in 0.9% sodium chloride at 5 mg/ml was administered intravesicularly once weekly for 6 weeks via sterile catheterization with a dwell time of 2 hours. Starting dose was 150 mg with a dose escalation model used until a maximal deliverable dose (MDD) of 500 mg/100 ml was achieved. Serum levels of Nab-paclitaxel was monitored 2 hours after voiding with high pressure liquid chromatography.

### Treatment assessment

The primary end point was dose-limiting toxicity (NCI common toxicity criteria grade 3 and 4 toxicity) and MDD; the secondary endpoint was response rate. Efficacy was evaluated by cytoscopy with biopsy, cytology, and CT imaging. Toxicities were graded according to the NCICTC 3.0. Systemic dose-limiting toxicity (DLT) were defined as grade 2, 3, 4 systemic toxicity. Localized DLT was defined as grade 3 and 4 hematuria, dysuria, urinary retension, urinary frequency/urgency, or bladder spasm. Follow-up evaluation was carried out at 12 weeks from first instillation with cystoscopy, biopsy, and urine cytology.

Table 1 shows phase I dose escalation (three patients treated at each dose) according to Fabonacci dose escalation design.

**Table 1**

| Dose-Level | *Nab*-paclitaxel (mg) | Final volume (mL) | Concentration (mg/ml) |
|---|---|---|---|
| D0 | 150 | 30 | 5.00 |
| D1 | 225 | 45 | 5.00 |
| D2 | 300 | 60 | 5.00 |
| D3 | 375 | 75 | 5.00 |
| D4 | 450 | 90 | 5.00 |
| D5 | 500 | 100 | 5.00 |

Table 2 shows patient demographics (No. of patients = 18).

**Table 2**

| Characteristic | Patients |
|---|---|
| Median age (range) | 71 (56-84) |
| Sex | |
| Male | 13 |
| Female | 5 |
| Inclusion | |
| Refused surgery | 16 |
| Medically inoperable | 2 |
| ECOG status | 18 |
| 0 | |
| Clinical stage | |
| Ta | 3 |
| Tis | 9 |
| T1 | 6 |
| Prior therapy with BCG | 18 |

Table 3 shows the treatment related NCICTC toxicities by dose level in 15 completed patients (all toxicities were grade 1)

**Table 3**

| | **150 mg** | **225 mg** | **300 mg** | **375 mg** | **450 mg** | **500 mg** |
|---|---|---|---|---|---|---|
| Dysuria | 0 | 1 | 1 | 1 | 0 | 2 |
| Urinary retension | 1 | 0 | 0 | 1 | 0 | 0 |
| Urinary frequency | 0 | 1 | 0 | 0 | 0 | 1 |
| Hematuria | 1 | 0 | 0 | 1 | 0 | 1 |

Twelve out of 18 patients (56%) experienced grade 1 local toxicities, with dysuria being the most common. No grade 2, 3, or 4 drug related local toxicities were encountered, and there was no apparent correlation between dose and toxicity. After 108 intravesical instillations, no systemic toxicity occurred.

There was no systemic absorption of *Nab*-paclitaxel up through the 375 mg dose. Among the 18 patients treated, serum level of *Nab*-paclitaxel was undetectable (<10 ng/ml) in 17/18 patients. One patient has 16.8 ng/ml at 450 mg dose, which was three magnitudes lower than the mean serum levels detected after standard intravenous administration. No grade 2 or greater toxicities attributed to administration of *Nab*-paclitaxel had occurred.

Table 4 shows individual clinical outcomes of 12-week cystoscopy and biopsy.

**Table 4**

| Patient # | Dose (mg) | Pretrial Stage | Response | Recurrent Stage |
|---|---|---|---|---|
| 1 | 150 | T1 | Complete | |
| 4 | 225 | Tis | Complete | |
| 5 | 225 | T1 | Complete | |
| 6 | 225 | Ta | Complete | |
| 10 | 375 | T1 | Complete | |
| 2 | 150 | Tis | No | Tis |
| 3 | 150 | Ta, Tis | No | Ta. Tis |
| 7 | 300 | Ta, Tis | No | Ta |
| 8 | 300 | Tis | No | T1, Tis |
| 9 | 300 | Ta, Tis | No | T1 |
| 11 | 375 | Tis | No | (no biopsy)* |
| 12 | 375 | T1 | No | T2 |
| 13 | 450 | T1, Tis | No | Ta, Tis |
| 14 | 450 | Tis | No | Tis |
| 15 | 450 | Ta | No | Tis |
| 16 | 500 | Ta | No | Ta |
| 17 | 500 | T1, Tis | No | T1, Tis |
| 18 | 500 | Tis | No | Tis |

| | | | | |
|---|---|---|---|---|
| *patient underwent cystectomy after developing visible papillary disease without cystoscopic biopsy. | | | | |

Five out of 18 evaluated patients had a complete response (negative biopsy and urine cytology). Of the 13 patients who recurred, one patient had evidence of stage progression at the post-treatment evaluation.

The experiment demonstrates that intravesical *Nab*-paclitaxel has had minimal local toxicity and systemic absorption and produces favorable response rates for treating refractory non-muscle-invasive bladder cancer.

### Example 2. Phase II study of single agent Nab-paclitaxel as second-line therapy in patients with metastatic urothelial carcinoma

This experiment demonstrates the effect of *Nab*-paclitaxel in patients with metastatic urothelial cancer failing first line cisplatin-based treatments for metastatic disease.

Patients with unresectable locally advanced or metastatic urothelial cancer of the bladder, ureter or renal pelvis, who progressed on or after first-line platinum based chemotherapy were enrolled on this open-label, two-stage, multicenter clinical trial. Key eligibility criteria included measurable disease with predominantly transitional cell histology, ECOG performance status 0-2, and adequate organ function. Patients treated with prior taxanes for metastatic disease, pre-existing neuropathy >= grade 1 or uncontrolled brain metastases or other illnesses were excluded.

### Key inclusion

Age ≥18 years of age
ECOG performance status ≤ 2
Locally advanced or metastatic disease
Histologically proven transitional cell carcinoma (mixed histologies permitted if TCC is MAJOR component)
Must have received one prior chemotherapeutic agent including a platinum (at least one cycle) for metastatic/recurrent disease
Neoadjuvant or adjuvant chemotherapy considered first line if patient progressed within 12 months of last dose
No prior taxanes for metastatic disease
Prior taxanes allowed in the adjuvant or neoadjuvant setting if >12 mo from last dose

### Key exclusion

Prior taxane for metastatic disease (or □ 12 months since neoadjuvant or adjuvant taxanes)
Pre-existing peripheral neuropathy □1 by NCI-CTC criteria.

Pregnant or lactating females.

Uncontrolled brain or leptomeningeal metastases(treated brain mets permitted if both known lesions and medications e.g. steroids for that indication are stable).

Serious or concurrent illness
History of class II-IV congestive heart failure.

Other malignancies except basal cell carcinoma of the skin or carcinoma in situ of the cervix or incidental prostate cancer (T1a, Gleason <7 PSA <10ng/ml) or any other tumor <5yrs prior to enrollment.
Investigational therapy or radiation therapy<30 days earlier
Patients not using adequate contraception

### Treatment

*Nab*-Paclitaxel (Abraxane®) 260-300mg/m2 intravenously every 3 weeks over 30 minutes. No maximum number of cycles, patients may be treated until disease progression, unacceptable toxicity, and/or voluntary withdrawal. No premedication was needed.

### Evaluation

Clinical evaluation, CBC and biochemistry were performed at baseline and before each cycle with CT scans of the chest, abdomen, and pelvis repeated every 2 cycles.

Pre treatment: history, physical exam, routine blood work within 7 days of study entry, CT chest, abdomen, pelvis and bone scan (if elevated alkaline phosphatase or bone pain), and brain imaging (as clinically indicated) within 28 days of study entry were obtained and evaluated.

On treatment: history, physical exam, routine blood work q3wks, CT chest, abdomen, pelvis q2months were evaluated.

### End points

The primary endpoint was RECIST-defined objective response. Responding patients had confirmatory CT scans performed at least one month following documentation of a response. Secondary endpoints were duration of response, disease control rate (stable disease >=16 weeks, PR or CR), PFS, overall survival, safety, and tolerability.

### Study design

*Nab*-paclitaxel is worthy of further study if response rate is >20%.

Two stage design: to test the null hypothesis that P<=0.050 versus the alternative that P>=0.200 has an expected sample size of 26.66 and a probability of early termination of 0.717.

If not effective, there is a 0.046 probability of concluding that it is (target for this value= 0.050). If effective, there is a 0.098 probability of concluding that it is not (target for this value = 0.100).

### Two-stage design

Stage 1: 21 pts. If >1 response proceed to stage 2.

Stage 2: Accrue an additional 20 pts.

Assuming a 10-15% drop out rate the total accrual will be 48-50 pts

### Demographics and accrual

Table 5 shows patient demographies.

**Table 5**

| | | |
|---|---|---|
| Total number of patients | | 50* |
| Median age (range) | | 67 (42-88) |
| Gender | M:F | 32:6 |
| Performance status (PS) | 0.1:2 | 13:18.6 |
| Histology | TCC:Mixed | 30:2 |
| Median cycles/pt | | 4(1-12) |
| Dose reduction | | 12/38 = 32% |

| | | |
|---|---|---|
| *not all patients are evaluable at the time of the experiment. | | |

### Preliminary response stage 1

Table 6 shows preliminary responses of patients treated with *Nab*-paclitaxel.

**Table 6**

| Best objective response | 29 pts evaluable | % |
|---|---|---|
| CR | 1 | 3% (CI 0.09-17.8%) |
| Partial response | 12 | 41% (CI 23.5-61%) |
| Stable disease | 9 | 31% (CI 15.3-50.8%) |
| CR+PR+SD | 22 | 76% (CI 56.5-89.7%) |
| Progressive disease | 7 | 24% (CI 10.3-43.5%) |

Over a 24 month period, accrual to this 2nd line metastatic TCC study was brisk (50 pts/5 institutions). *Nab*-Paclitaxel was generally well tolerated, and showed preliminary ORR of 44% (13/29) and a disease control rate (CR+PR+SD) of 76% (22/29).

### Example 3A: A phase II study of single-agent Nab-paclitaxel in platinum-refractory second-line metastatic urothelial carcinoma (UC).

In this multi-institutional phase II study, the efficacy and tolerability of *Nab*-paclitaxel as a single agent was evaluated in patients with platinum-refractory metastatic UC.

### Methods

Patients with measurable UC, progressing on or after first-line platinum-based chemotherapy were enrolled onto this two-stage trial. ABI-007 was given at 260 mg/m² IV q3weekly until progression. Clinical evaluation, CBC and blood chemistries were performed every cycle with restaging CT scans every 2 cycles.

### Results

48 patients were enrolled with the following baseline characteristics: Male: Female 40:8; median age 68; ECOG Performance Status 0:1:2, 15:24:8. 248 cycles were delivered with a median of 5.5 cycles/pt with 17/48 pts (35%) requiring dose reductions. Most frequent adverse events (AE) were alopecia (12%), fatigue (12%), pain (12%), neuropathy (9%) and nausea (4%). The most frequent grade 3+ AE were pain (45%), hypertension (14%), fatigue (8%), joint stiffness (5%), neuropathy (4%) and weakness (4%).

Forty patients were evaluable for response: 1 (2.5%) had a complete response (CR), 11 (28%) had a partial responses (PR), 9 (23%) had stable disease (SD) and 20 (49%) jad progressive disease. One patient was inevaluable for response, 7 patients were too early for evaluation.

Single-agent ABI-007 was well tolerated with a response rate (CR+PR) of 33% (12/36) and a clinical benefit rate (CR+PR+SD) of 58% (21/36), representing one of the highest reported response rates to date in the second-line UC setting.

### Example 3B: A phase II study of single-agent Nab-paclitaxel in platinum-refractory second-line metastatic urothelial carcinoma (UC).

The primary objective of this study was to evaluate the efficacy using tumor response rate (ORR) of *Nab*-paclitaxel in second line UC patients. The secondary objective was to evaluate the disease control rate (DCR), progression-free-survival (PFS), overall survival (OS) and safety and tolerability.

### Methods

Patients with measurable disease with a performance status of 0-2, progressing on or after first-line platinum-based chemotherapy were enrolled onto this two-stage trial. *Nab-*paclitaxel was given at 260 mg/m² IV q3weekly until progression, intolerable toxicity or voluntary withdrawal. Physical and blood tests were performed every three weeks and CT scans of the chest/abdomen/pelvis were performed every 2 cycles.

### Key inclusion criteria

Histologically proven TCC of the urinary tract
Measureable distant or unresectable local disease
Progression on or ≤ 1 year of platinum-based chemotherapy
Performance Status 0-2
Renal function (GFR>40 ml/min)
Taxane naive

### Results

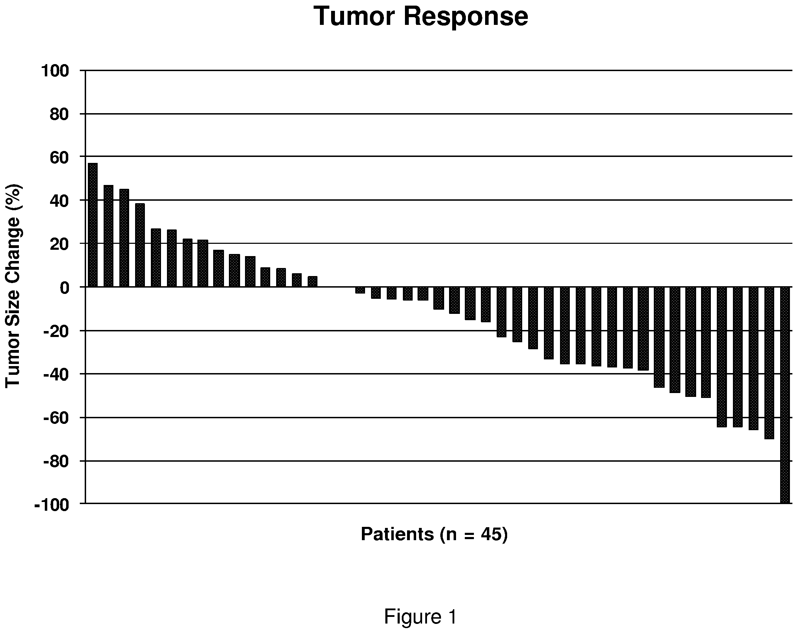

48 patients were enrolled and their main baseline characteristics and treatment exposure are summarized in Tables 7A and 7B. Figure 1 shows the percentage of change in tumor size in 45 patients. Partial response (PR) was achieved by 15 patients (32%), and stable disease (SD) was observed in 10 patients (21%), resulting in a disease control rate (DCR=PR+SD) of 25 patients (53%). 22 patients (47%) had disease progression, and one patient was not evaluable (Table 8).

**Table 7A: Main characteristics at baseline and treatment exposure**

| | | |
|---|---|---|
| N | 48 | |
| Age | 66 (39-88) | |
| Gender | Male 40 Female 8 | |
| Performance Status | PS | 0 16pts |
| | | 1 24pts |
| | | 2 8pts |
| Histology | TCC 43 Mixed 2 Missing 3 | |
| Hemoglobin (g/L) | 120 (range 86-155) | |
| Visceral/Bone Metastases | Yes 73% No 27% | |
| Median time from last chemo | 5.2 mo (range 0.69-49 months) | |
| Prior platinum response | Yes 53% No 47% | |
| Median No. of cycles | 6.0 cycles | |
| Median (range) | 1-15 cycles | |
| Dose Reductions* | 16/48 (33%) | |

| | | |
|---|---|---|
| *Most commonly due to fatigue or neuropathy | | |

**Table 7B**

| Best Objective Response | 47* Evaluable n (%) | 95% CI |
|---|---|---|
| Partial Response | 15 (32%) | 19.09% - 47.12% |
| Stable Disease | 10 (21%) | 10.70% - 35.66% |
| Disease Control Rate (PR+SD) | 25 (53%) | 38.08% - 67.89% |
| Progressive Disease | 22 (47%) | 32.11% - 61.92% |

| | | |
|---|---|---|
| * 1 patient inevaluable | | |

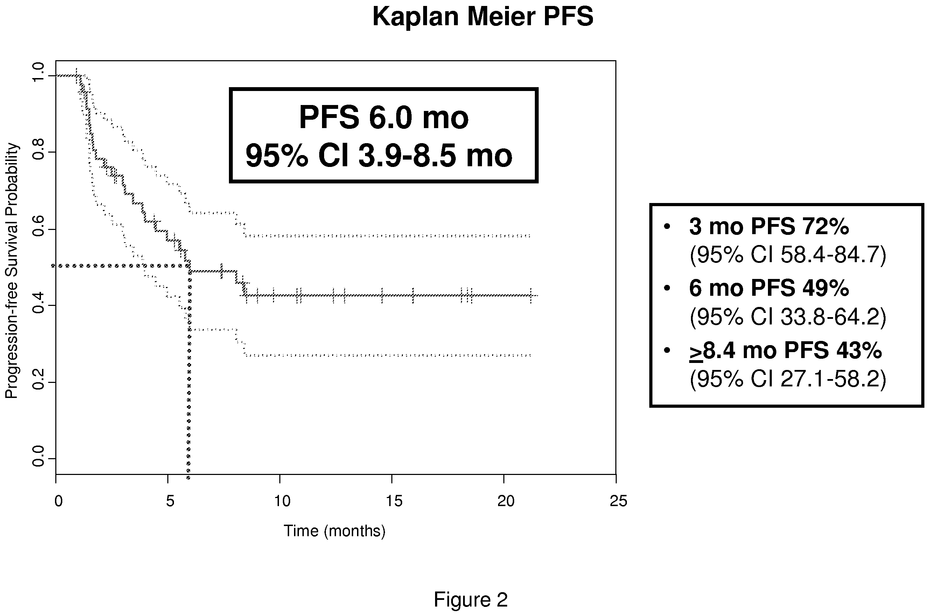

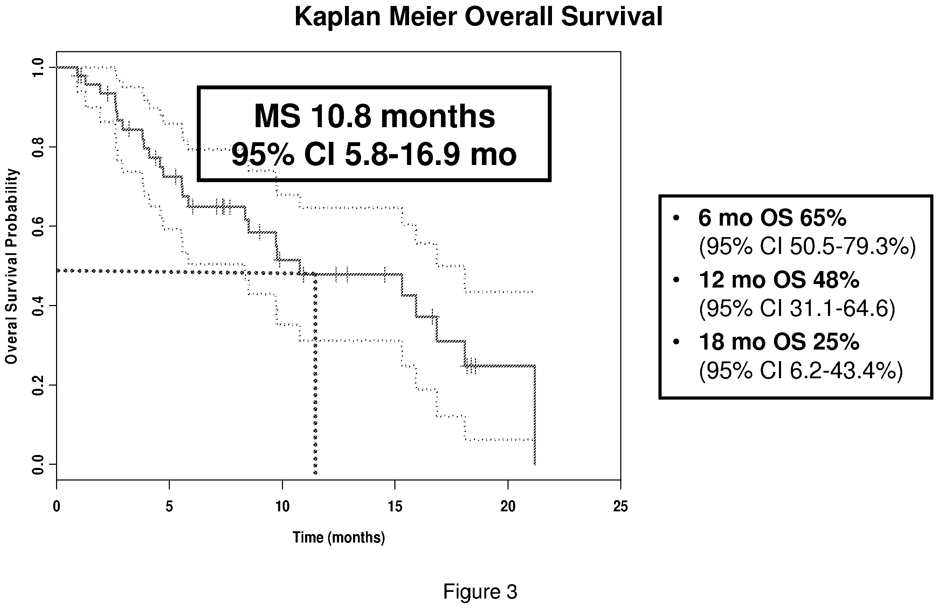

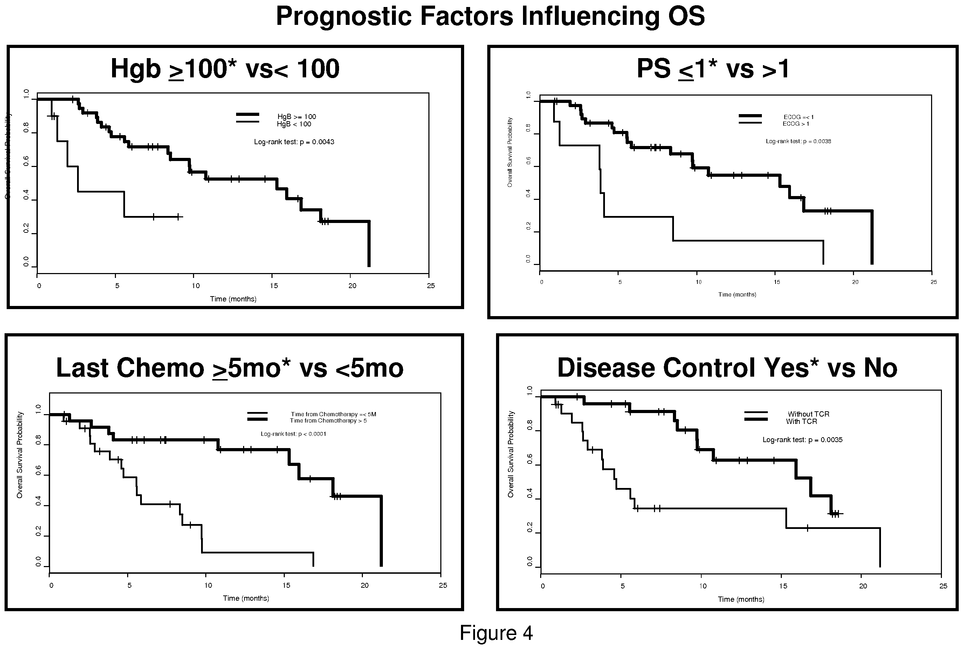

As secondary objectives, progression free survival (PFS) and overall survival (OS) were evaluated. Progression free survival was 6 months (95% CI 3.9-8.5 mo; Figure 2), and overall survival (OS) was 10.8 (94% CI 5.8 - 16.9 mo; Figure 3). In addition, prognostic factors influencing overall survival were evaluated. Hemoglobin ≥ 100 g/L, Performance Status (PS) of ≤ 1, more than 5 months from chemotherapy and presence of disease control showed a positive effect on overall survival (Figure 4). The most common overall toxicities, toxicities 3+ and reasons to discontinue treatment are summarized in Tables 8 to 10.

**Table 8: Most common overall toxicities**

| Adverse event | Total 1380 Incidence n (%) | Grade 3+ n (%) |
|---|---|---|
| Alopecia | 177 (13%) | - |
| Fatigue | 171 (12%) | 11 (6%) |
| Pain | 170 (12%) | 46 (27%) |
| Neuropathy | 132 (10%) | 5 (1%) |
| Dyspnea | 45 (3%) | 3 (7%) |
| Edema | 44 (3%) | - |
| Flu-like Symptoms | 39 (3%) | - |
| Diarrhea | 36 (3%) | - |
| Anorexia | 35 (3%) | 1 (3%) |

**Table 9: Toxicity grade 3+**

| Total 107 events (7.8%) | n | % |
|---|---|---|
| Pain | 46 | 43% |
| Fatigue | 11 | 10% |
| Hypertension | 7 | 7% |
| Joints Stiffness | 7 | 7% |
| Neuropathy | 5 | 5% |
| Weakness | 5 | 5% |
| Dyspnea | 3 | 3% |

**Table 10: Off treatment reasons**

| Reason | n (%) |
|---|---|
| Progressive Disease | 25 (56%) |
| Toxicity* | 9 (16%) |
| Consent Withdrawal | 7 (11%) |
| Death | 2 (4%) |
| Radiation of target lesion | 1 (2%) |
| Intercurrent Illness | 1 (2%) |
| Other | 3 (9%) |

| | |
|---|---|
| * 7 due to neuropathy in cycle 3 (2), 6, 7, 9, 10, 14 | |

### Conclusion

In summary, *Nab*-paclitaxel was well tolerated and showed the highest single agent response rates in second line UC. The ORR was 32 % and the DCR was 53% in this study. Progression free survival was 6 months and overall survival was 10.8 months. Main toxicities were fatigue, pain and neuropathy. Furthermore, PS status, hemoglobin levles, time from last chemotherapy and ability to achieve disease control have emerged as important prognostic markers for overall survival.

### Example 4A: A phase II trial of neoadjuvant ABI-007, carboplatin, and gemcitabine (ACG) in patients with locally advanced carcinoma of the bladder.

ABI-007, carboplatin and gemcitabine combination therapy was studied in patients with locally advanced carcinoma of the bladder. The primary study endpoint was the proportion of patients with pCR at cystectomy.

### Methods

Eligible patients had T₂₋₄,N₀,M₀ or T_{any},N₁₋₃,M₀ bladder cancer with ECOG PS 0-1, and adequate marrow (granulocyte count > 1,500/mm³, platelet > 100,000/mm³, and hemoglobin > 9.0 g/dl), hepatic (transaminases < 2.5 X upper limit of normal, alkaline phosphatase < 2.5 X upper limit of normal, and bilirubin < 1.5 mg/dl) and renal function (serum creatinine < 2.0 mg/dl and/or creatinine clearance > 40 ml/min). Patients were treated with intravenous ABI-007 260 mg/m² and carboplatin (target area under the curve=5) on day 1 and with gemcitabine 800 mg/m² on days 1 and 8, followed by radical cystectomy after three cycles of therapy.

### Results

27 patients have been enrolled to date. By clinical staging, 20 patients had T2 disease, 5 had T3, 2 had T4 disease, and 2 had nodal enlargement. All patients were evaluable for toxicity and 22 patients were evaluable for response. Three patients were excluded from evaluation for response due to a change in dose schedule, 1 patient was excluded due to refusal of cystectomy and 1 patient was excluded due to withdrawal from the study. 25/27 patients received all three cycles (78 total cycles) with doses reduced in 26 cycles for toxicity. All patients had transient grade 3-4 neutropenia and 17 patients received filgrastim, but only two had febrile neutropenia. Six patients had pCR with an additional 5 having residual carcinoma in situ (CIS) and 1 with T₁ disease at cystectomy. 54% of evaluable patients had no muscle invasive disease at cystectomy.

### Conclusion

Neoajuvant ACG is active in bladder cancer with a pCR rate nearing 30% and nearly as many patients with CIS but no residual invasive disease. Marrow toxicity is significant but manageable.

### Example 4B: A phase II trial of neoadjuvant ABI-007, carboplatin, and gemcitabine (ACG) in patients with locally advanced carcinoma of the bladder.

The primary objective of this open-label study with Minimax two-stage accrual design was to estimate the rate of pathologic complete response (pCR) following three cycles of neoadjuvant ACG in patients with muscle invasive urothelial carcinoma.

### Eligibility criteria

T₂₋₄,N₀,M₀ or T_{any},N₁₋₃,M₀ bladder cancer
ECOG PS 0-1
Hematology: granulocyte count ≥ 1,500/mm3, platelet count ≥ 100,000/mm3, and hemoglobin ≥ 9.0 g/dl
Liver: AST & ALT ≤ 2.5 X ULN, alk phos ≤ 2.5 X ULN, and bilirubin ≤ 1.5 mg/dl
Renal: creatinine ≤ 2.0 mg/dl and/or creatinine clearance ≥ 40 ml/min

### Methods

Patients were treated with ABI-007 260 mg/m² given as a 30 min IV infusion and carboplatin (target area under the curve=5) given as a 15 min IV infusion on day 1 and with gemcitabine 800 mg/m² given as a 30 min IV infusion on days 1 and 8.

### Results

27 patients have been enrolled to date and their baseline characteristics are summarized in Table 11. 25/27 patients received all three cycles of therapy (78 total cycles) with doses reduced in 26 cycles. All patients had grade 3-4 neutropenia, 17 patients received GCSF (filgrastin). 2 patients had episodes of febrile neutropenia. Other common toxicities were alopecia (27) and neuropathy (5). 6 patients showed a pathologic complete response (27% of evaluable patients) and 5 patients with residual carcinoma in situ (pCIS), 1 patient had pT1. 11/22 patients (50%) had no residual muscle invasive disease.

**Table 11:**

| | |
|---|---|
| Enrolled (N) | 27 |
| Evaluable (N) | 22* |
| Median Age (range) | 66 (38-82) |
| Male/Female | 21/6 |
| Performance status (ECOG) | |
| 0 | 16 |
| 1 | 11 |
| Clinical Stage | 18 |
| T2N0 | 1 |
| T2N1 | 1 |
| T2N2 | 5 |
| T3N0 | 2 |
| T4N0 | |

| | |
|---|---|
| *3-change in schedule,1-refused cystectomy, 1-withdrew | |

### Conclusion

ACG was well-tolerated with transient neutropenia as most common toxicity. Pathologic complete response was observed in 27% of evaluable patients to date. No residual muscle invasive disease was found in 50% of patients.

## Claims

1. A composition comprising nanoparticles comprising a taxane and albumin for use in a method of treating bladder cancer in an individual, wherein the method further comprises administering a platinum-based agent and gemcitabine.

2. The composition for use of claim 1, wherein the method comprises concurrent administration of the composition, the platinum-based agent and gemcitabine to the individual.

3. The composition for use of claim 1 or 2, wherein the bladder cancer is platinum-refractory bladder cancer.

4. The composition for use of any one of claims 1 to 3, wherein the bladder cancer is locally advanced bladder cancer.

5. The composition for use of any one of claims 1 to 4, wherein the bladder cancer is muscle-invasive bladder cancer.

6. The composition for use of any one of claims 1 to 5, wherein the bladder cancer is urothelial carcinoma.

7. The composition for use of any one of claims 1 to 5, wherein the bladder cancer is a high grade bladder cancer.

8. The composition for use of any one of claims 1 to 5, wherein the bladder cancer is metastatic bladder cancer.

9. The composition for use of any one of claims 1 to 8, wherein the platinum-based agent is carboplatin.

10. The composition for use of claim 9, wherein the method comprises administering the carboplatin intravenously at AUC of about 5.

11. The composition for use of any one of claims 1 to 10, wherein the method comprises administering gemcitabine intravenously at about 800 mg/m².

12. The composition for use of any one of claims 1 to 11, wherein the method comprises administering the nanoparticle composition intravenously.

13. The composition for use of claim 12, wherein the nanoparticle composition is administered at about 260-300 mg/m².

14. The composition for use of any one of claims 1 to 13, wherein the taxane is paclitaxel.

15. The composition for use of any one of claims 1 to 14, wherein the nanoparticles in the composition have an average diameter of no greater than about 200 nm, preferably less than about 200 nm.

16. The composition for use of any one of claims 1 to 15, wherein the taxane in the nanoparticles is coated with albumin.

## Patentansprüche

1. Zusammensetzung umfassend Nanopartikel, die ein Taxan und Albumin umfassen, für die Verwendung in einem Verfahren zur Behandlung von Blasenkrebs bei einem Individuum, wobei das Verfahren weiter das Verabreichen eines auf Platin basierenden Mittels und Gemcitabin umfasst.

2. Zusammensetzung für die Verwendung nach Anspruch 1, wobei das Verfahren die gleichzeitige Verabreichung der Zusammensetzung, des auf Platin basierenden Mittels und Gemcitabin an das Individuum umfasst.

3. Zusammensetzung für die Verwendung nach Anspruch 1 oder 2, wobei der Blasenkrebs Platin-refraktärer Blasenkrebs ist.

4. Zusammensetzung für die Verwendung nach einem der Ansprüche 1 bis 3, wobei der Blasenkrebs lokal fortgeschrittener Blasenkrebs ist.

5. Zusammensetzung für die Verwendung nach einem der Ansprüche 1 bis 4, wobei der Blasenkrebs Muskel-invasiver Blasenkrebs ist.

6. Zusammensetzung für die Verwendung nach einem der Ansprüche 1 bis 5, wobei der Blasenkrebs ein urotheliales Karzinom ist.

7. Zusammensetzung für die Verwendung nach einem der Ansprüche 1 bis 5, wobei der Blasenkrebs ein High-grade Blasenkrebs ist.

8. Zusammensetzung für die Verwendung nach einem der Ansprüche 1 bis 5, wobei der Blasenkrebs metastatischer Blasenkrebs ist.

9. Zusammensetzung für die Verwendung nach einem der Ansprüche 1 bis 8, wobei das auf Platin basierende Mittel Carboplatin ist.

10. Zusammensetzung für die Verwendung nach Anspruch 9, wobei das Verfahren das intravenöse Verabreichen bei AUC von ungefähr 5 umfasst.

11. Zusammensetzung für die Verwendung nach einem der Ansprüche 1 bis 10, wobei das Verfahren das intravenöse Verabreichen von Gemcitabin mit ungefähr 800 mg/m² umfasst.

12. Zusammensetzung für die Verwendung nach einem der Ansprüche 1 bis 11, wobei das Verfahren das intravenöse Verabreichen der Nanopartikelzusammensetzung umfasst.

13. Zusammensetzung für die Verwendung nach Anspruch 12, wobei die Nanopartikelzusammensetzung mit ungefähr 260-300 mg/m² verabreicht wird.

14. Zusammensetzung für die Verwendung nach einem der Ansprüche 1 bis 13, wobei das Taxan Paclitaxel ist.

15. Zusammensetzung für die Verwendung nach einem der Ansprüche 1 bis 14, wobei die Nanopartikel in der Zusammensetzung einen mittleren Durchmesser von nicht größer als ungefähr 200 nm, bevorzugt weniger als ungefähr 200 nm haben.

16. Zusammensetzung für die Verwendung nach einem der Ansprüche 1 bis 15, wobei das Taxan in den Nanopartikeln mit Albumin beschichtet ist.

## Revendications

1. Composition comprenant des nanoparticules qui comprennent un taxane et de l'albumine pour une utilisation dans un procédé de traitement du cancer de la vessie chez un individu, dans laquelle le procédé comprend en outre l'administration d'un agent à base de platine et de gemcitabine.

2. Composition pour une utilisation selon la revendication 1, dans laquelle le procédé comprend l'administration simultanée de la composition, de l'agent à base de platine et de la gemcitabine à l'individu.

3. Composition pour une utilisation selon la revendication 1 ou 2, dans laquelle le cancer de la vessie est un cancer de la vessie réfractaire au platine.

4. Composition pour une utilisation selon l'une quelconque des revendications 1 à 3, dans laquelle le cancer de la vessie est un cancer de la vessie localement avancé.

5. Composition pour une utilisation selon l'une quelconque des revendications 1 à 4, dans laquelle le cancer de la vessie est un cancer de la vessie invasif du muscle.

6. Composition pour une utilisation selon l'une quelconque des revendications 1 à 5, dans laquelle le cancer de la vessie est le carcinome urothélial.

7. Composition pour une utilisation selon l'une quelconque des revendications 1 à 5, dans laquelle le cancer de la vessie est un cancer de la vessie de grade élevé.

8. Composition pour une utilisation selon l'une quelconque des revendications 1 à 5, dans laquelle le cancer de la vessie est un cancer de la vessie métastatique.

9. Composition pour une utilisation selon l'une quelconque des revendications 1 à 8, dans laquelle l'agent à base de platine est le carboplatine.

10. Composition pour une utilisation selon la revendication 9, dans laquelle le procédé comprend l'administration du carboplatine par voie intraveineuse à une AUC d'environ 5.

11. Composition pour une utilisation selon l'une quelconque des revendications 1 à 10, dans laquelle le procédé comprend l'administration de la gemcitabine par voie intraveineuse à environ 800 mg/m².

12. Composition pour une utilisation selon l'une quelconque des revendications 1 à 11, dans laquelle le procédé comprend l'administration de la composition de nanoparticules par voie intraveineuse.

13. Composition pour une utilisation selon la revendication 12, dans laquelle la composition de nanoparticules est administrée à environ 260 à 300 mg/m².

14. Composition pour une utilisation selon l'une quelconque des revendications 1 à 13, dans laquelle le taxane est le paclitaxel.

15. Composition pour une utilisation selon l'une quelconque des revendications 1 à 14, dans laquelle les nanoparticules dans la composition ont un diamètre moyen de pas plus d'environ 200 nm, de préférence de moins d'environ 200 nm.

16. Composition pour une utilisation selon l'une quelconque des revendications 1 à 15, dans laquelle le taxane dans les nanoparticules est revêtu avec de l'albumine.
